# EUROPEAN PATENT SPECIFICATION

(11) **EP 2 367 425 B1**
(45) Date of publication and mention of the grant of the patent: **28.02.2018**
(21) Application number: 09832656.4
(22) Date of filing: 11.12.2009
(51) Int. Cl.: A61K 31/337, A61K 31/517, A61K 31/661, A61K 31/7064, A61K 45/06, B82Y 5/00, A61P 35/00, A61K 47/42, A61K 9/51, A61K 31/675

(54) **Combination therapy including a taxane and a further therapeutic agent**
Kombinationstherapie enhaltend eine taxane und ein weiteres therapeutisches Mittel.
Thérapie combinatoire avec un taxane et un agent thérapeutique supplémentaire

(30) Priority: 11.12.2008 US 201624 P; 14.05.2009 US 178430 P
(43) Date of publication of application: 28.09.2011
(73) Proprietor: Abraxis BioScience, LLC, Los Angeles, CA 90025 (US)
(72) Inventor: TRIEU, Vuong, Agoura Hills, CA 91301 (US); D'CRUZ, Osmond, Los Angeles CA 90025 (US); DESAI, Neil, P., Pacific Palisades CA 90272 (US)
(74) Representative: Weber, Martin
(86) International application number: PCT/US2009/067766
(87) International publication number: WO 2010/068925

(56) References cited:
- US-A1- 2003 008 924
- US-A1- 2006 263 434
- US-A1- 2006 275 305
- US-A1- 2008 161 251
- DUDEK ARKADIUSZ Z ET AL: "Safety of nab-paclitaxel plus sunitinib: analysis of three cases.", ANTICANCER RESEARCH 2008 SEP-OCT LNKD- PUBMED:19031964, vol. 28, no. 5B, September 2008 (2008-09), pages 3099-3105, XP002683570, ISSN: 0250-7005
- VOLK LISA D ET AL: "Nab-paclitaxel efficacy in the orthotopic model of human breast cancer is significantly enhanced by concurrent anti-vascular endothelial growth factor A therapy.", NEOPLASIA (NEW YORK, N.Y.) JUN 2008 LNKD- PUBMED:18516298, vol. 10, no. 6, June 2008 (2008-06), pages 613-623, XP002683571, ISSN: 1476-5586
- MEALY N E ET AL: "Drugs under development for the treatment of head and neck cancer", DRUGS OF THE FUTURE, PROUS SCIENCE, ES, vol. 31, no. 7, 1 July 2006 (2006-07-01), pages 625-639, XP008086555, ISSN: 0377-8282
- HUI SUN ET AL: "Co-administration of perifosine with paclitaxel synergistically induces apoptosis in ovarian cancer cells: More than just AKT inhibition", CANCER LETTERS, NEW YORK, NY, US, vol. 310, no. 1, 12 June 2011 (2011-06-12) , pages 118-128, XP028258237, ISSN: 0304-3835, DOI: 10.1016/J.CANLET.2011.06.010 [retrieved on 2011-06-29]

## Description

### RELATED APPLICATIONS

This application claims priority benefit to provisional applications 61/201,624, filed on December 11, 2008 and 61/178,430, filed on May 14, 2009.

### TECHNICAL FIELD

The present invention relates to compositions for use in the treatment of proliferative diseases comprising the administration of a combination of a taxane and at least one other therapeutic agent useful in the treatment of proliferative diseases. In particular, the invention relates to the use of nanoparticles comprising paclitaxel and albumin (such as Abraxane®) in combination with other agents or radiation, which may be used for the treatment of cancer.

### BACKGROUND

The failure of a significant number of tumors to respond to drug and/or radiation therapy is a serious problem in the treatment of cancer. In fact, this is one of the main reasons why many of the most prevalent forms of human cancer still resist effective chemotherapeutic intervention, despite certain advances in the field of chemotherapy.

Cancer is now primarily treated with one or a combination of three types of therapies: surgery, radiation, and chemotherapy. Surgery is a traditional approach in which all or part of a tumor is removed from the body. Surgery generally is only effective for treating the earlier stages of cancer. While surgery is sometimes effective in removing tumors located at certain sites, for example, in the breast, colon, and skin, it cannot be used in the treatment of tumors located in other areas, inaccessible to surgeons, nor in the treatment of disseminated neoplastic conditions such as leukemia. For more than 50% of cancer individuals, by the time they are diagnosed they are no longer candidates for effective surgical treatment. Surgical procedures may increase tumor metastases through blood circulation during surgery. Most of cancer individuals do not die from the cancer at the time of diagnosis or surgery, but rather die from the metastasis and the recurrence of the cancer.

Other therapies are also often ineffective. Radiation therapy is only effective for individuals who present with clinically localized disease at early and middle stages of cancer, and is not effective for the late stages of cancer with metastasis. Radiation is generally applied to a defined area of the subject's body which contains abnormal proliferative tissue, in order to maximize the dose absorbed by the abnormal tissue and minimize the dose absorbed by the nearby normal tissue. However, it is difficult (if not impossible) to selectively administer therapeutic radiation to the abnormal tissue. Thus, normal tissue proximate to the abnormal tissue is also exposed to potentially damaging doses of radiation throughout the course of treatment. There are also some treatments that require exposure of the subject's entire body to the radiation, in a procedure called "total body irradiation", or "TBI." The efficacy of radiotherapeutic techniques in destroying abnormal proliferative cells is therefore balanced by associated cytotoxic effects on nearby normal cells. Because of this, radiotherapy techniques have an inherently narrow therapeutic index which results in the inadequate treatment of most tumors. Even the best radiotherapeutic techniques may result in incomplete tumor reduction, tumor recurrence, increasing tumor burden, and induction of radiation resistant tumors.

Chemotherapy involves the disruption of cell replication or cell metabolism. Chemotherapy can be effective, but there are severe side effects, e.g., vomiting, low white blood cells (WBC), loss of hair, loss of weight and other toxic effects. Because of the extremely toxic side effects, many cancer individuals cannot successfully finish a complete chemotherapy regime. Chemotherapy-induced side effects significantly impact the quality of life of the individual and may dramatically influence individual compliance with treatment. Additionally, adverse side effects associated with other agents are generally the major dose-limiting toxicity (DLT) in the administration of these drugs. For example, mucositis is one of the major dose limiting toxicity for several anticancer agents, including the antimetabolite cytotoxic agents 5-FU, methotrexate, and antitumor antibiotics, such as doxorubicin. Many of these chemotherapy-induced side effects if severe may lead to hospitalization, or require treatment with analgesics for the treatment of pain. Some cancer individuals die from the chemotherapy due to poor tolerance to the chemotherapy. The extreme side effects of anticancer drugs are caused by the poor target specificity of such drugs. The drugs circulate through most normal organs of individuals as well as intended target tumors. The poor target specificity that causes side effects also decreases the efficacy of chemotherapy because only a fraction of the drugs is correctly targeted. The efficacy of chemotherapy is further decreased by poor retention of the anti-cancer drugs within the target tumors.

Due to the severity and breadth of neoplasm, tumor and cancer, there is a great need for effective treatments of such diseases or disorders that overcome the shortcomings of surgery, chemotherapy, and radiation treatment.

Paclitaxel has been shown to have significant antineoplastic and anticancer effects in drug-refractory ovarian cancer and has shown excellent antitumor activity in a wide variety of tumor models, and also inhibits angiogenesis when used at very low doses (Grant et al., Int. J. Cancer, 2003). The poor aqueous solubility of paclitaxel, however, presents a problem for human administration. Indeed, the delivery of drugs that are inherently insoluble or poorly soluble in an aqueous medium can be seriously impaired if oral delivery is not effective. Accordingly, currently used paclitaxel formulations (e.g., Taxol®) require a Cremophor® to solubilize the drug. The presence of Cremophor® in this formulation has been linked to severe hypersensitivity reactions in animals (Lorenz et al., Agents Actions 7:63-67 (1987)) and humans (Weiss et al., J. Clin. Oncol. 8:1263-68 (1990)) and consequently requires premedication of individuals with corticosteroids (dexamethasone) and antihistamines. It was also reported that clinically relevant concentrations of the formulation vehicle Cremophor® EL in Taxol® nullify the antiangiogenic activity of paclitaxel, suggesting that this agent or other anticancer drugs formulated in Cremophor® EL may need to be used at much higher doses than anticipated to achieve effective metronomic chemotherapy (Ng et al., Cancer Res., 64:821-824 (2004)). As such, the advantage of the lack of undesirable side effects associated with low-dose paclitaxel regimes vs. conventional MTD chemotherapy may be compromised. See also U.S. Patent Pub. No. 2004/0143004; WO00/64437.

It has been found that nanoparticle compositions of a taxane (such as albumin bound paclitaxel (*nab*-paclitaxel or Abraxane®)) have significantly lower toxicities than other taxanes like Taxol® and Taxotere® with significantly improved outcomes in both safety and efficacy.

Combination chemotherapy, e.g., combining one or more other agents or other modes of treatment, e.g., combining for example, chemotherapy with radiation or surgery and chemotherapy, have been found to be more successful than single agent chemotherapeutics or individual modes of treatment respectively.

Other references include U.S. Pub. No. 2006/0013819; U.S. Pub. No. 2006/0003931; 20060263434, and PCT Application Nos. WO05/117986; WO05/117978; WO05/000900, WO06/089290, WO08/057562, WO2009126938A1, WO2009126401A1, WO2009126175A1.

More effective treatments for proliferative diseases, especially cancer, are needed.

### BRIEF SUMMARY OF THE INVENTION

The invention provides combination therapy for use in methods of treating a proliferative disease (such as cancer), comprising administering to the individual a) an effective amount of a composition comprising nanoparticles comprising a taxane and a carrier protein (such as albumin), and b) an effective amount of at least one other agent that inhibits a prosurvival and/or inflammatory signal. According to the invention and for the purpose of this application, such agent is an agent selected from the group consisting of Akt inhibitor, inhibitor of bcl-2, inhibitor of bcl-xl and inhibitor of both bcl-2 and bcl-xl. In some embodiments, the invention relates to treating a proliferative disease (such as cancer) in an individual comprising administering to the individual a) an effective amount of a composition comprising nanoparticles comprising paclitaxel coated with an albumin (such as Abraxane®), and b) an effective amount of at least one other agent that inhibits a prosurvival and/or inflammatory signal. In some embodiments, the agent affects the signaling pathway involving Akt. In some embodiments, the agent affects the signaling pathway involving bcl-2. In some embodiments, the agent inhibits taxane-mediated pro-survival and/or inflammatory response. In some embodiments, the agent abrogates or reduces stress response elicited by taxane. In some embodiments, the proliferative disease is resistant to the treatment of taxane when administered alone.

In some embodiments, the composition comprising nanoparticles (also referred to as "nanoparticle composition") and the other agent are administered simultaneously, either in the same composition or in separate compositions. In some embodiments, the nanoparticle composition and the other agent are administered sequentially, i.e., the nanoparticle composition is administered either prior to or after the administration of the other agent. In some embodiments, the nanoparticle composition is administered prior to the administration of the other agent.

In some embodiments, the administration of the nanoparticle composition and the other agent are concurrent, i.e., the administration period of the nanoparticle composition and that of the other agent overlap with each other. In some embodiments, the nanoparticle composition is administered for at least one cycle (for example, at least any of 2, 3, or 4 cycles) prior to the administration of the other agent. In some embodiments, the other agent is administered for at least any of one, two, three, or four weeks after the termination of the nanoparticle composition.

In some embodiments, the administration of the nanoparticle composition and the other agent are non-concurrent. For example, in some embodiments, the administration of the nanoparticle composition is terminated before the other agent is administered. In some embodiments, the administration of the other agent is terminated before the nanoparticle composition is administered.

In some embodiments, the other agent is an inhibitor of Akt, including for example perifosine, GSK690693, triciribine phosphate monohydrate, API-2/TCN, XL418, and erlotinib (Tarceva®). In some embodiments, the agent is perifosine. In some embodiments, the agent is in an amount effective to suppress taxane-mediated upregulation of Akt *in vivo.*

For example, in some embodiments, the method of treating a proliferative disease (such as cancer), comprises administering to the individual a) an effective amount of a composition comprising nanoparticles comprising a taxane (such as paclitaxel) and a carrier protein (such as albumin), and b) an effective amount of a perifosine. In some embodiments, the perifosine is in an amount effective to suppress taxane-mediated upregulation of Akt *in vivo.* in some embodiments, there is provided a method of treating a proliferative disease (such as cancer), comprising administering to the individual a) an effective amount of a composition comprising nanoparticles comprising a taxane (such as paclitaxel) and a carrier protein (such as albumin), and b) an effective amount of an ertolinib. In some embodiments, the erlotinib is in an amount effective to suppress taxane-mediated upregulation of Akt *in vivo.*

Further described herein is an inhibitor of MAP kinase, including for example SL327, U0126, SP600125, PD98059, SB203580, SB202190, Arctigenin, PD198306, PD254552,PD318894, and PD320125-2. The inhibitor may be in an amount effective to suppress taxane-mediated upregulation of MAP kinase *in vivo.*

In some embodiments, the other agent is an inhibitor of bcl-2, including for example HA 14-1, Obatoclax, ABT-737, and ABT263. Other agents that inhibit bcl-2 are described in US20090099072, WO06082304A2, and US7459434.

For example, in some embodiments, the method of treating a proliferative disease (such as cancer) in an individual comprises administering to the individual a) an effective amount of a composition comprising nanoparticles comprising a taxane and a carrier protein, and b) an effective amount of a small molecule that inhibits the activity of bcl-2. In some embodiments, method of treating a proliferative disease (such as cancer) in an individual comprising administering to the individual a) an effective amount of a composition comprising nanoparticles comprising paclitaxel and albumin (such as Abraxane®, and b) an effective amount of a small molecule that inhibits the activity of bcl-2.

In some embodiments, the agent is in an amount effective to suppress taxane-mediated upregulation of bcl-2 *in vivo.* In some embodiments, the other agent is an antisense oligonucleotide that inhibits the expression of bcl-2. In some embodiments, the antisense oligonucleotide is an antisense oligodeoxynucleotide. In some embodiments, the antisense oligonucleotide is an antisense oligodeoxyribonucleotide. In some embodiments, the other agent is oblimersen. For example, in some embodiments, the method of treating a proliferative disease (such as cancer, for example melanoma, e.g., advanced melanoma) in an individual comprises administering to the individual a) an effective amount of a composition comprising nanoparticles comprising paclitaxel coated with an albumin (such as Abraxane®), and b) an antisense oligonucleotide that inhibits the expression of bcl-2. In some embodiments, the method of treating a proliferative disease (such as cancer, for example melanoma, e.g., advanced melanoma) in an individual comprises administering to the individual: a) an effective amount of a composition comprising nanoparticles comprising paclitaxel coated with an albumin (such as Abraxane®), and b) oblimersen.

In some embodiments, the other agent is an inhibitor of bcl-xL. In some embodiments, the other agent is an inhibitor of both bcl-2 and bcl-xL.

In some embodiments, the method further comprises administering to the individual an alkylating agent, such as temozolomide. Thus, for example, in some embodiments, the method of treating a proliferative disease (such as cancer, for example melanoma, e.g., advanced melanoma) in an individual comprises administering to the individual: a) an effective amount of a composition comprising nanoparticles comprising paclitaxel coated with an albumin (such as Abraxane®), and b) an antisense oligonucleotide that inhibits the expression of bcl-2 (such as oblimersen), and c) an alkylating agent (such as temozolomide). In some embodiments, the method of treating melanoma (such as advanced melanoma) in an individual comprises administering to the individual: a) an effective amount of nanoparticles comprising paclitaxel coated with albumin (such as Abraxane®), b) an effective amount of oblimersen, and c) an effective amount of temozolomide. In some embodiments, the individual has normal LDH.

Further described herein is an inhibitor of IL-6, including for example Tocilizumab, Am-80, IL-6 receptor fusion protein, Gp130, SAMURAI. The inhibitor may be in an amount effective to suppress taxane-mediated upregulation of IL-6 *in vivo.*

Further described herein is an inhibitor of IL-8, including for example IL-8INH, [Ala-IL-8]77, REPARIXIN, Ascidian Lissoclinum sp, lissoclinum disulfoxide. The inhibitor may be in an amount effective to suppress taxane-mediated upregulation of IL-8 *in vivo.*

Further described herein is an inhibitor of TNF-a, including for example Adalimumab, Certolizumab pegol, Etanercept, Infliximab, and TNF inhibitor. The inhibitor may be in an amount effective to suppress taxane-mediated upregulation of TNF-a *in vivo.*

Further described herein is an inhibitor of NF-κB (such as inhibitor of the phosphorylation of NF-κB p65). The inhibitor may be in an amount effective to suppress taxane-mediated upregulation of NF-κB (such as inhibitor of NF-κB p65 phosphorylation) *in vivo.* Inhibitors of NF-kB can be found, for example, at http://people.bu.edu/gilmore/nf-kb/inhibitors/index.html, incorporated herein in its entirety.

Further described herein is an inhibitor of the p50 (such as inhibitor of p50 phosphorylation). The inhibitor may be in an amount effective to suppress taxane-mediated upregulation of p50 (such as p50 phophorylation) *in vivo.*

Further described herein is an inhibitor of p42/44 (such as inhibitor of p42/44 phosphorylation), including for example PD98059, U0126. The inhibitor may be in an amount effective to suppress taxane-mediated upregulation of p42/44 (such as p42/44 phosphorylation) *in vivo.*

The methods of the invention generally comprise administration of a composition comprising nanoparticles comprising a taxane and a carrier protein. In some embodiments, the nanoparticle composition comprises nanoparticles comprising paclitaxel and an albumin. In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 400, including for example about 20 to about 200 nm, about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane (such as paclitaxel) coated with a carrier protein, such as albumin (e.g., human serum albumin). In some embodiments, the composition comprises taxane in both nanoparticle and non-nanoparticle form, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the nanoparticle composition is substantially free (such as free) of surfactants (such as Cremophor®, Tween 80, or other organic solvents used for the administration of taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and taxane in the nanoparticle composition is about 18:1 or less, such as about 15:1 or less, for example about 10:1 or less. In some embodiments, the weight ratio of carrier protein (such as albumin) and taxane in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and taxane in the nanoparticle portion of the composition is about any one of 1:2, 1:3, 1:4, 1:5, 1:10, 1:15, or less.

In some embodiments, the particle composition comprises one or more of the above characteristics.

In some embodiments, the nanoparticle composition is Abraxane®. Nanoparticle compositions comprising other taxanes (such as docetaxel and ortataxel) may also comprise one or more of the above characteristics.

Also disclosed are kits and compositions useful for methods described herein.

The composition for use of the present application are useful for the treatment of various diseases, including, for example, breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

These and other aspects and advantages of the present invention will become apparent from the subsequent detailed description and the appended claims. It is to be understood that one, some, or all of the properties of the various embodiments described herein may be combined to form other embodiments of the present invention.

### BRIEF DESCRIPTION OF FIGURES

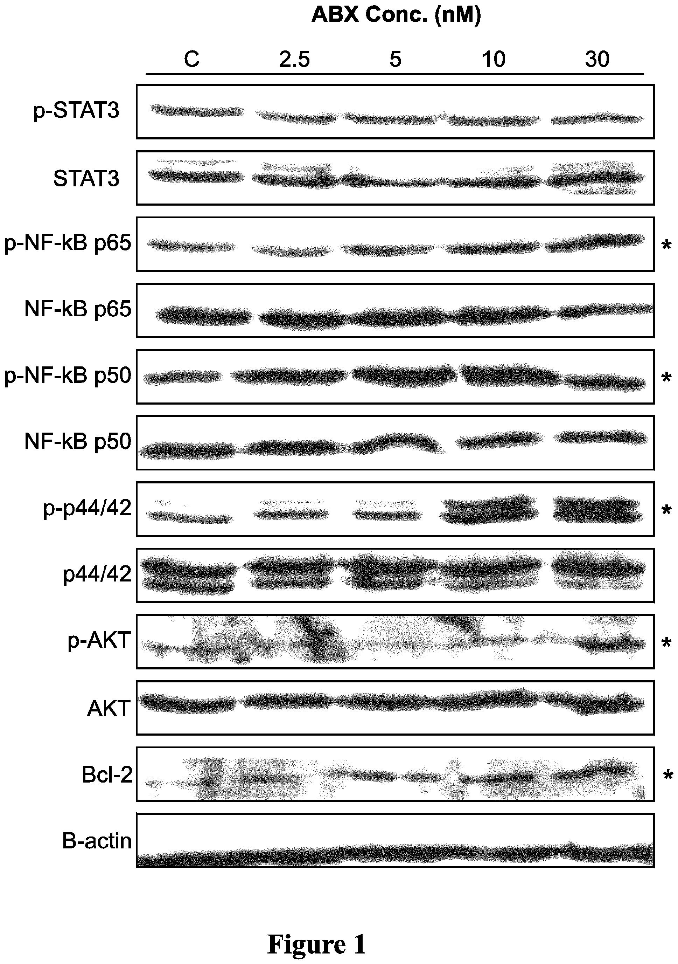

Figure 1 shows increased pro-survival signals in response to low level Abraxane® in MDAMB-231 cells. Increased signal indicated by *. Levels of β-actin were used as loading control.

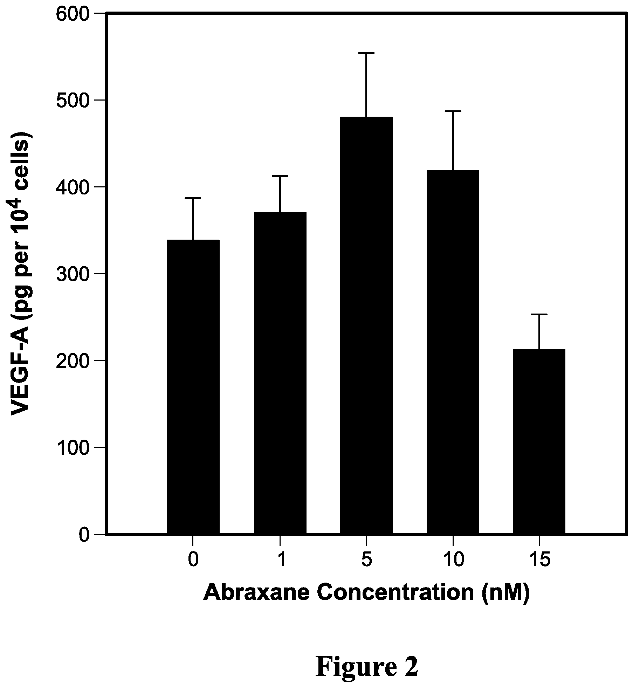

Figure 2 shows increased secretion of VEGF in response to low level Abraxane® in MDA-MB-231 cells as measured by ELISA.

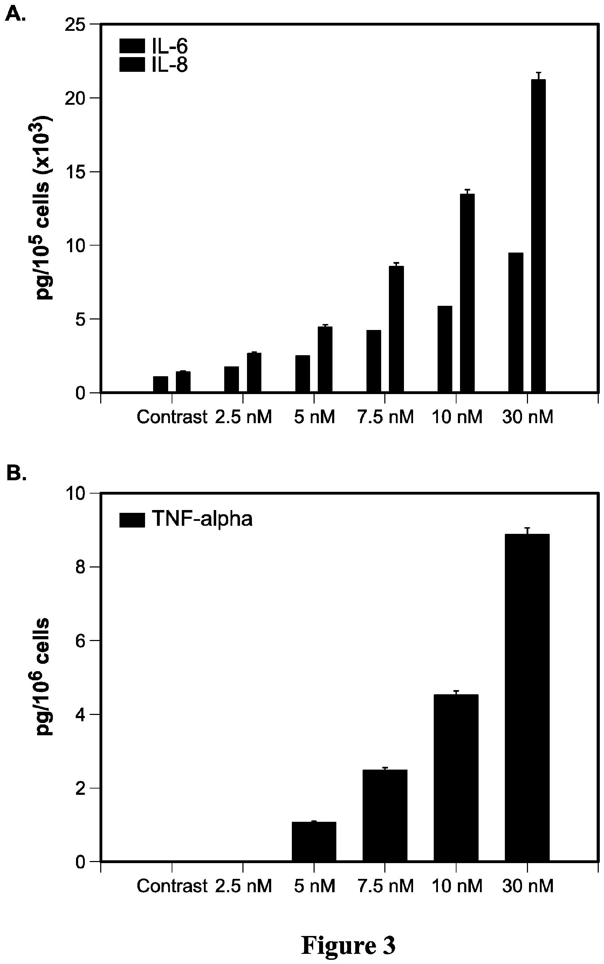

Figure 3 shows increased secretion of inflammatory proteins in MDA-MB-231 cells in response to low level Abraxane®. A. Increased secretion of IL-6 and IL-8; B. Increased secretion of TNF-a.

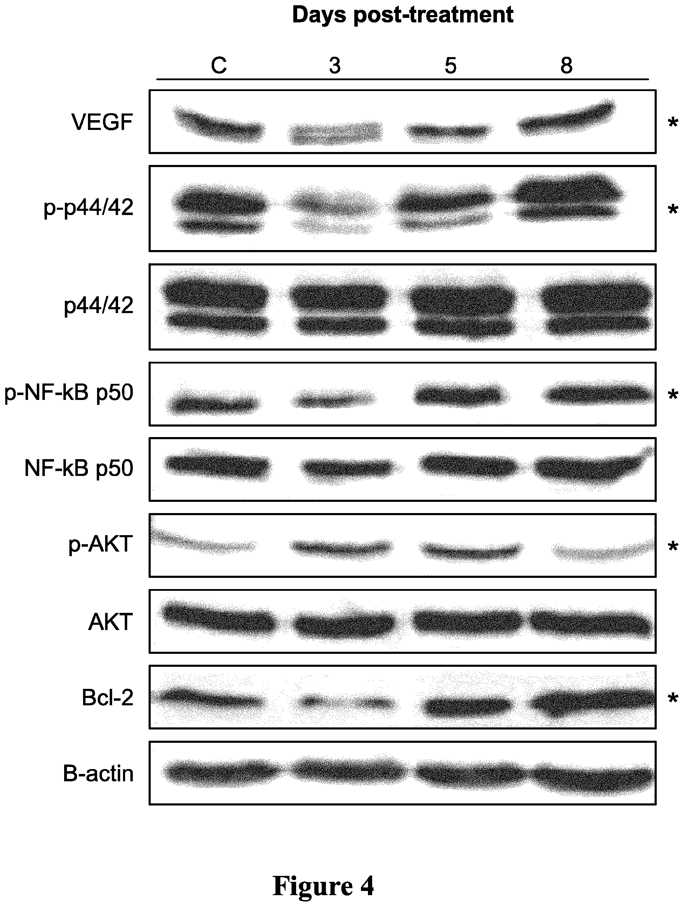

Figure 4 shows upregulation of pro-survival signals *in vivo* in the remaining MDA-MB-231 breast tumors that survived Abraxane® treatment. Increased signal indicated by *. Levels of β- actin were used as loading control.

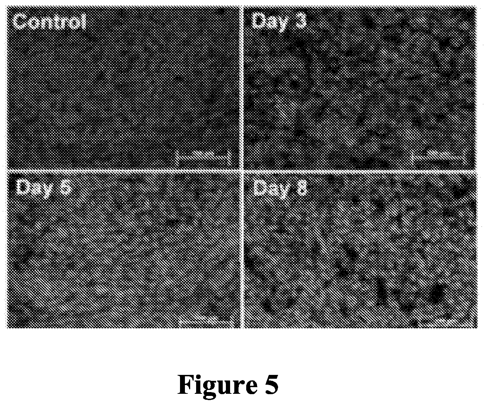

Figure 5 shows increased bcl-2 expression *in vivo* in the surviving MDAMB-231 breast tumors following Abraxane® treatment. Animals were sacrificed on the indicated days post-treatment and tumor sections were stained for bcl-2 expression.
Figure 6A shows Western blots of lysates of 231-Luc⁺ cells treated with a *nab*-paclitaxel (0-30 nM) for 8 (left), 24 (center) and 48 (right) hours. Control and treated 231-Luc⁺ cell lysates were analyzed by Western blot for changes in the phosphorylated and non-phosphorylated forms of NF-κB p50, NF-κB p65, Erk p44/42, Akt, and unmodified Bcl-xL. β-actin served as a loading control. The asterisks represent targets with significant changes in response to *nab*-paclitaxel treatment.
Figures 6B and 6C show production of inflammatory cytokines (IL-6 and IL-8 in Figure 6B and TNF-alpha in Figure 6C) by 231-Luc⁺ cells treated for 72 hours with *nab*-paclitaxel (0-30 nM) determined in the conditioned medium by Luminex assay. Data are presented as the mean cytokine concentration normalized per number of viable tumor cells that derived from two independent experiments ± SE.
Figure 7A shows Western blots of mixed lysates from tumors harvested on days 3, 5 and 8. Western blot analysis of expression of the phosphorylated and non-phosphorylated forms of NF-κB p50, p44/42, Akt, and unmodified Bcl-2 and Bcl-xLwere analyzed. β-actin was used as a loading control.
Figure 7B shows tumor sections from mice treated with nab-paclitaxel for 3, 5, and 8 days stained with antibodies against pro-survival proteins, Bcl-2 (top row) and Bcl-xL (bottom row). All images were acquired at 200X magnification from the margins of the tumor.

### DETAILED DESCRIPTION OF THE INVENTION

The present invention provides combination therapy comprising a first therapy comprising administration of nanoparticles comprising a taxane and a carrier protein (such as albumin) in conjunction with a second agent that inhibits a prosurvival and/or inflammatory signal, for use in a method of treating a proliferative disease.

The present invention is based on the finding that paclitaxel in a nanoparticle formulation, specifically, Abraxane® (or *nab*-paclitaxel) elicits an upregulation of pro-survival and inflammatory signals such as Akt, VEGF-A, bcl-2, bcl-xL, IL-6, IL-8, TNF-a, as well as phosphorylated NF-κB p65, p50, and p42/44 kinases. We hypothesize that combination therapy of a nanoparticle composition comprising a taxane and a carrier protein (such as Abraxane®) with inhibitors of pro-survival and inflammatory signals would significantly improve the efficacy of nanoparticle forms of taxane-based therapy by counteracting the stress responses in therapy-spared tumor cells.

The present application thus provides combination therapy. It is to be understood by a person of ordinary skill in the art that the combination therapy methods described herein requires that one agent or composition be administered in conjunction with another agent. "In conjunction with" refers to administration of one treatment modality in addition to another treatment modality, such as administration of a nanoparticle composition described herein in addition to administration of the other agent to the same individual. As such, "in conjunction with" refers to administration of one treatment modality before, during or after delivery of the other treatment modality to the individual.

The methods described herein are generally useful for treatment of diseases, particularly proliferative diseases. As used herein, "treatment" is an approach for obtaining beneficial or desired clinical results. For purposes of this invention, beneficial or desired clinical results include, but are not limited to, any one or more of: alleviation of one or more symptoms, diminishment of extent of disease, preventing or delaying spread (e.g., metastasis, for example metastasis to the lung or to the lymph node) of disease, preventing or delaying recurrence of disease, delay or slowing of disease progression, amelioration of the disease state, and remission (whether partial or total). Also encompassed by "treatment" is a reduction of pathological consequence of a proliferative disease. The methods of the invention contemplate any one or more of these aspects of treatment.

As used herein, a "proliferative disease" is defined as a tumor disease (including benign or cancerous) and/or any metastases, wherever the tumor or the metastasis are located, more specifically a tumor selected from the group comprising one or more of (and in some embodiments selected from the group consisting of) breast cancer, genitourinary cancer, lung cancer, gastrointestinal cancer, epidermoid cancer, melanoma, ovarian cancer, pancreatic cancer, neuroblastoma, colorectal cancer, head and neck cancer. In some embodiments, the cancer is selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma. In a broader sense of the invention, a proliferative disease may furthermore be selected from hyperproliferative conditions such as hyperplasias, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty. In some embodiments, the proliferative disease is cancer. In some embodiments, the proliferative disease is a non-cancerous disease. In some embodiments, the proliferative disease is a benign or malignant tumor. Where hereinbefore and subsequently a tumor, a tumor disease, a carcinoma or a cancer are mentioned, also metastasis in the original organ or tissue and/or in any other location are implied alternatively or in addition, whatever the location of the tumor and/or metastasis is.

The term "effective amount" used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay recurrence. An effective amount can be administered in one or more administrations. In the case of cancer, the effective amount of the drug or composition may: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; and/or (vii) relieve to some extent one or more of the symptoms associated with the cancer.

In some embodiments, the invention relates to treating a primary tumor. In some embodiments, it relates to treating metastatic cancer (that is, cancer that has metastasized from the primary tumor). In some embodiments, it relates to treating a proliferative disease (such as cancer) (and in broader aspect method of treating a proliferative disease) at advanced stage(s). In some embodiments, it relates to treating breast cancer (which may be HER2 positive or HER2 negative), including, for example, advanced breast cancer, stage IV breast cancer, locally advanced breast cancer, and metastatic breast cancer. In some embodiments, it relates to treating lung cancer, including, for example, non-small cell lung cancer (NSCLC, such as advanced NSCLC), small cell lung cancer (SCLC, such as advanced SCLC), and advanced solid tumor malignancy in the lung. In some embodiments, it relates to treating any of ovarian cancer, head and neck cancer, gastric malignancies, melanoma (including metastatic melanoma and malignant melanoma), colorectal cancer, pancreatic cancer, and solid tumors (such as advanced solid tumors). In some embodiments, it relates to treating a disease that is refractory to treatment of a taxane when administered alone. In some embodiments, it relates to reducing cell proliferation and/or cell migration. In some embodiments, it relates to treating any of the following diseases: restenosis, stenosis, fibrosis, angiogenesis, psoriasis, atherosclerosis, and proliferation of smooth muscle cells. The present invention also relates to delaying development of any of the proliferative diseases described herein.

The term "individual" is a mammal, including humans. An individual includes, but is not limited to, human, bovine, horse, feline, canine, rodent, or primate. In some embodiments, the individual is human. The individual (such as human) may have advanced disease or lesser extent of disease, such as low tumor burden. In some embodiments, the individual is at an early stage of a proliferative disease (such as cancer). In some embodiments, the individual is at an advanced stage of a proliferative disease (such as an advanced cancer). In some embodiments, the individual is HER2 positive. In some embodiments, the individual is HER2 negative.

The methods may be practiced in an adjuvant setting. "Adjuvant setting" refers to a clinical setting in which an individual has had a history of a proliferative disease, particularly cancer, and generally (but not necessarily) been responsive to therapy, which includes, but is not limited to, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high risk" or "low risk") depends upon several factors, most usually the extent of disease when first treated. The methods provided herein may also be practiced in a neoadjuvant setting, i.e., the method may be carried out before the primary/definitive therapy. In some embodiments, the individual has previously been treated. In some embodiments, the individual has not previously been treated. In some embodiments, the treatment is a first line therapy.

It is understood that aspect and embodiments of the invention described herein include "consisting" and/or "consisting essentially of" aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

### Methods of combination therapy

The present invention relates to treating a proliferative disease (such as cancer) in an individual, comprising administering to the individual: a) an effective amount of a composition comprising nanoparticles comprising a taxane and a carrier protein (such as albumin); and b) an effective amount of at least one other agent that inhibits a prosurvival and/or inflammatory signal. The present invention relates to treating a proliferative disease (such as cancer) in an individual, comprising administering to the individual: a) an effective amount of a composition comprising nanoparticles comprising a taxane and a carrier protein (such as albumin); and b) an effective amount of at least one other agent that inhibits a prosurvival and/or inflammatory signal, wherein the other agent is in an amount effective to suppress taxane-mediated upregulation of the prosurvival and/or inflammatory signal. In some embodiments, the taxane is any of (and in come embodiments consisting essentially of) paclitaxel, docetaxel, and ortataxel. In some embodiments, the nanoparticle composition comprises Abraxane®. In some embodiments, the proliferative disease is a cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple mveloma.

In some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of at least one other agent that inhibits a prosurvival and/or inflammatory signal, wherein the nanoparticle composition and the other agent are administered concurrently. In some embodiments, the administrations of the nanoparticle composition and the other agent are initiated at about the same time (for example, within any one of 1, 2, 3, 4, 5, 6, or 7 days). In some embodiments, the administrations of the nanoparticle composition and the other agent are terminated at about the same time (for example, within any one of 1, 2, 3, 4, 5, 6, or 7 days). In some embodiments, the administration of the other agent continues (for example for about any one of 0.5, 1, 2, 3, 4, 5, 6, 7, 8, 9, 10, 11, or 12 months) after the termination of the administration of the nanoparticle composition. In some embodiments, the administration of the other agent is initiated after (for example after about any one of 0.5, 1, 2, 3, 4, 5, 6, 7, 8, 9, 10, 11, or 12 months) the initiation of the administration of the nanoparticle composition. In some embodiments, the administrations of the nanoparticle composition and the other agent are initiated and terminated at about the same time. In some embodiments, the administrations of the nanoparticle composition and the other agent are initiated at about the same time and the administration of the other agent continues (for example for about any one of 0.5, 1, 2, 3, 4, 5, 6, 7, 8, 9, 10, 11, or 12 months) after the termination of the administration of the nanoparticle composition. In some embodiments, the administration of the nanoparticle composition and the other agent stop at about the same time and the administration of the other agent is initiated after (for example after about any one of 0.5, 1, 2, 3, 4, 5, 6, 7, 8, 9, 10, 11, or 12 months) the initiation of the administration of the nanoparticle composition.

In some embodiments, the taxane is any of (and in some embodiments consisting essentially of) paclitaxel, docetaxel, and ortataxel. In some embodiments, the taxane is paclitaxel. In some embodiments, the taxane is docetaxel. In some embodiments, the nanoparticle composition comprises Abraxane®. In some embodiments, the nanoparticle composition is Abraxane®.

Thus, for example, in some embodiments, the invention relates to treating a proliferative disease (such as cancer) in an individual, comprising administering to the individual: a) an effective amount of a composition comprising nanoparticles comprising a taxane (such as paclitaxel) coated with a carrier protein (such as albumin); and b) an effective amount of at least one other agent that inhibits a prosurvival and/or inflammatory signal. In some embodiments, the nanoparticles have an average size of 20-400 nm, such as 40-200 nm. In some embodiments, the method of treating a proliferative disease (such as cancer) in an individual comprises administering to the individual: a) an effective amount Abraxane®; and b) an effective amount of at least one other agent that inhibits a prosurvival and/or inflammatory signal. In some embodiments, the nanoparticle composition (such as Abraxane®) and the other agent are administered concurrently. In some embodiments, the proliferative disease is a cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

In some embodiments, the other agent affects the signaling pathway involving Akt. In some embodiments, the agent affects the signaling pathway involving bcl-2 and/or bcl-xL. In some embodiments, the agent inhibits taxane-mediated prosurvival and/or inflammatory response.

The other agents described herein can be the agents themselves, pharmaceutically acceptable salts thereof, and pharmaceutically acceptable esters thereof, as well as steroisomers, enantiomers, racemic mixtures, and the like. The other agent or agents as described can be administered as well as a pharmaceutical composition containing the agent(s), wherein the pharmaceutical composition comprises a pharmaceutically acceptable carrier vehicle, or the like.

Reference to an agent herein applies to the other agent or its derivatives and accordingly the invention contemplates and includes either of these embodiments (agent; agent or derivative(s)). "Derivatives" or "analogs" of an agent or other chemical moiety include, but are not limited to, compounds that are structurally similar to the other agent or moiety or are in the same general chemical class as the other agent or moiety. In some embodiments, the derivative or analog of the other agent or moiety retains similar chemical and/or physical property (including, for example, functionality) of the other agent or moiety.

Thus, in some embodiments, the invention relates to treating a proliferative disease (such as cancer) in an individual, comprising administering to the individual a) an effective amount of a composition comprising nanoparticles comprising a taxane and a carrier protein (e.g., albumin) and b) an effective amount of at least one other agent that affects the PI3K/Akt pathway and/or cAMP/AMPK pathway. Because these pathways are interrelated, an agent that affects one signaling pathway frequently affects the other pathway (either directly or indirectly).

In some embodiments, the invention relates to treating a proliferative disease (such as cancer) in an individual, comprising administering to the individual a) an effective amount of a composition comprising nanoparticles comprising a taxane and a carrier protein (e.g., albumin) and b) an effective amount of at least one other agent that inhibits PI3K/Akt activation. In some embodiments, the proliferative disease is cancer. In some embodiments, the cancer is any of HER2+ breast cancer, chronic mylogenous leukemia CML, ovarian cancer, endometrial cancer, sarcoma, SCCHN (squamous cell carcinomaterm of the head and neck), and thyroid cancer.

The PI3K/Akt signaling pathway described herein includes any members or components that directly or indirectly participate in the signal transduction cascade. These include, but are not limited to, PI3 kinase, Akt, mTOR, PDK1, RAPTOR (regulatory associated protein if mTOR), TSC1 (tuberous sclerosis complex 1), TSC2, PTEN (phosphatase and tenesin homolog), and downstream effectors such as Bax, bcl-2, MDM2, p53, XIAP,S6,GSK-3,IKKs, cyclin D, HIF1, HIF2, Glut1, LAT1, and c-Myc. Components of the PI3/Akt signaling pathway may also include RHEB, Rictor, S6K, 4EBP1, cAMP, cAMPK, GβL, IRS, PIP2, PIP3, Rho, Ras, Abl, PKC, eIF4E, PDGFR, VEGFR, and VHL. The agent that affects (such as inhibits) the PI3K/Akt signaling pathway can thus act through modulation of any one or more of these components.

Thus, a method of treating a proliferative disease (such as cancer) in an individual, may comprise administering to the individual: a) an effective amount of a composition comprising nanoparticles comprising a taxane and a carrier protein (such as albumin); and b) an effective amount of an agent that inhibits PI3 kinase (PI3K). Suitable inhibitors of PI3K include, but are not limited to, wortmannin and the derivatives or analogs thereof; celecoxib and analogs thereof, such as OSU-03012 and OSU-03013; 3-deoxy-D-myo-inositol analogs, such as PX-316; 2'-substituted 3'-deoxy-phosphatidyl-myo-inositol analogs; fused heteroaryl derivatives; 3-(imidazo[1,2-a]pyridin-3-yl) derivatives; Ly294002; quinazoline-4-one derivatives, such as IC486068; 3-(hetero)aryloxy substituted benzo(b)thiophene derivatives; viridins, including semi-synthetic viridins such as such as PX-866 (acetic acid (1S, 4E, 10R, 11R, 13S, 14R)-[4-dially laminomethylene-6-hydroxy-1-methoxymethyl-10,13-dimethy 1-3,7,17-trioxo-1,3,4,7,10, 11,12,13,14,15,16,17-dodecahydro-2-oxa-cyclopenta[a]phenanthren-11-yl ester); and wortmannin and derivatives thereof.

In some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of an agent that inhibits Akt kinase (such as Akt1, Akt2, and/or Akt3). In some embodiments, the method of treating a proliferative disease (such as cancer) in an individual comprises administering to the individual: a) an effective amount of a composition comprising nanoparticles comprising a taxane and a carrier protein (such as albumin); and b) an effective amount of and agent that inhibits (1) recruitment of Akt to the cell membrane; (2) activation by PDK1 or PDK2, (3) substrate phosphorylation; and/or (4) one of the downstream target of Akt.

In some embodiments, the other agent inhibits phosphorylation of S473 of the human Akt kinase, but not T308. In some embodiments, the second compound inhibits phosphorylation of T308 of the human Akt kinase, but not S473. In some embodiments, the other agent inhibits phorphorylation of both S473 and T308 of the Akt kinase. In some embodiments, the other agent interferes with the membrane localization of the Akt kinase. Suitable inhibitors of Akt kinase include, but are not limited to, Akt-1-1 (inhibits Akt1), Akt-1-1,2 (inhibits Akt1 and 2), API-59CJ-Ome, 1-H-imidazo[4,5-c]pyridinyl compounds, indole-3-carbinol and derivatives thereof, perifosine, phosphatidylinositol ether lipid analogues, triciribine (TCN or API-2 or NCI identifier: NSC 154020).

In some embodiments, the other agent is perifosine. Thus, for example, in some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of perifosine. In some embodiments, the method of treating a proliferative disease (such as cancer) in an individual comprises administering to the individual: a) an effective amount of a composition comprising nanoparticles comprising paclitaxel coated with an albumin (such as Abraxane®); and b) an effective amount of perifosine. In some embodiments, the taxane is in an amount that is effective to suppress taxane-mediated upregulation of Akt. In some embodiments, the proliferative disease is cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

In some embodiments, the method of treating a proliferative disease (such as cancer) in an individual comprises administering to the individual: a) an effective amount of a composition comprising nanoparticles comprising a taxane and a carrier protein (such as albumin); and b) an effective amount of perifosine, wherein the nanoparticle composition is administered intravenously, wherein the perifosine is administered orally. In some embodiments, the method of treating a proliferative disease (such as cancer) in an individual comprises administering to the individual: a) an effective amount of a composition comprising nanoparticles comprising paclitaxel coated with an albumin (such as Abraxane®); and b) an effective amount of perifosine. In some embodiments, the nanoparticle composition and the perifosine are administered concurrently. In some embodiments, the proliferative disease is selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

In some embodiments, the method of treating a proliferative disease (such as cancer) in an individual comprises administering to the individual: a) an effective amount of a composition comprising nanoparticles comprising a taxane (such as paclitaxel) and a carrier protein (such as albumin), wherein the taxane is in the dosage range of about 60-300 mg/m²; and b) about 10-500 mg/day (including for example about 20-100, such as 50 mg/day) or about 1-150 mg/kg/day perifosine. In some embodiments, the method of treating a proliferative disease (such as cancer) in an individual comprises administering to the individual: a) an effective amount of a composition comprising nanoparticles comprising paclitaxel coated with an albumin (such as Abraxane®), wherein the taxane is in the dosage range of about 60-300 mg/m²; and b) about 10-500 mg/day (including for example about 20-100, such as 50 mg/day) or about 1-150 mg/kg/day perifosine. In some embodiments, the nanoparticle composition is administered intravenously. In some embodiments, the perifosine is administered orally. In some embodiments, the administrations of the drugs are concurrent. breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

The methods described herein include methods of administering perifosine. In some embodiments, the method comprises administering two or more compositions selected from a nanoparticle composition comprising a taxane and a carrier protein, perifosine, and an agent other than perifosine. Thus, the present application in some embodiments relates to treating a proliferative disease (such as cancer) in an individual, comprising administering to the individual in addition to the nanoparticle composition, a) an effective amount of perifosine, and b) an effective amount of an agent other than perifosine.

The agent other than perifosine in some embodiment can be an antimetabolite, antimetabolite agent (such as a nucleoside analog, including for example purine analogs and pyrimidine analogs). An "antimetabolic agent" is an agent which is structurally similar to a metabolite, but cannot be used by the body in a productive manner. Many antimetabolite agents interfere with production of nucleic acids, RNA and DNA. For example, the antimetabolite can be a nucleoside analog, which includes, but is not limited to, azacitidine, azathioprine, capecitabine (Xeloda®), cytarabine, cladribine, cytosine arabinoside (ara-C, cytosar), doxifluridine, fluorouracil (such as 5-fluorouracil), UFT, hydoxyurea, gemcitabine, mercaptopurine, methotrexate, thioguanine (such as 6-thioguanine). Other anti-metabolites include, for example, L-asparaginase (Elspa), decarbazine (DTIC), 2-deoxy-D-glucose, and procarbazine (matulane). In some embodiments, the nucleoside analog is any of (and in some embodiments selected from the group consisting of) gemcitabine, fluorouracil, and capecitabine. In some embodiments, the method is for treatment of metastatic breast cancer or locally advanced breast cancer. In some embodiments, the method is for first line treatment of metastatic breast cancer. In some embodiments, the method is for treatment of breast cancer in a neoadjuvant setting. In some embodiments, the method is for treatment of any of NSCLC, colorectal cancer, pancreatic cancer, multiple myeloma, or advanced solid tumor.

In some embodiments, the agent other than perifosine is selected from the group consisting of capecitabine, bortezomib, and imatinib.

Thus, for example, in some embodiments, the invention relates to treating colorectal cancer (such as colon cancer), comprising administering to the individual: a) an effective amount of a composition comprising nanoparticles comprising a taxane and a carrier protein (such as albumin); and b) an effective amount of perifosine. In some embodiments, the method of treating colorectal cancer comprises administering to the individual: a) an effective amount of a composition comprising nanoparticles comprising a taxane and a carrier protein (such as albumin); b) an effective amount of perifosine, and c) an effective amount of an antimetabolite (such as capecitabine). In some embodiments, the method comprises administering about 500-2000 mg/m² capecitabine. Randomized phase II study of perifosine in combination with capecitabine versus capecitabine alone in patients with second or third-line metastatic colon cancer showed that perifosine in combination with capecitabine more than doubled median time to progression over capecitabine alone in patients with advanced, metastatic colon cancer. Vukelja S et al., J Clin Oncol 27:15s, 2009 (suppl; abstract 4081) (2009 ASCO Annual Meeting).

Other combinations involving perifosine are also contemplated. For example, in some embodiments, the invention relates to treating myeloma, comprising administering to the individual: a) an effective amount of a composition comprising nanoparticles comprising a taxane and a carrier protein (such as albumin); b) an effective amount of perifosine, and c) an effective amount of bortezomib.

In some embodiments, the invention relates to treating gastrointestinal stromal tumor, comprising administering to the individual: a) an effective amount of a composition comprising nanoparticles comprising a taxane and a carrier protein (such as albumin); b) an effective amount of perifosine, and c) an effective amount of imatinib. In some embodiments, the gastrointestinal stromal tumor is resistant to the treatment with imatinib alone. Phase II study of perifosine plus imatinib for patients with imatinib-resistant gastrointestinal stromal tumor shows that the additional of perifosine to imatinib has minimal activity in imatinib-refractory gastrointestinal stromal tumor. Conley AP et al., J Clin Oncol 27:15s, 2009 (suppl; abstract 10563) (2009 ASCO Annual Meeting).

Also disclosed herein are methods of treating renal cell carcinoma in an individual by administering to the individual an effective amount of perifosine. In some examples, there is disclosed a method of treating renal cell carcinoma, comprising administering to the individual an effective amount of perifosine. Phase II studies of perifosine in metastatic or advanced renal cell carcinoma progressing after prior therapy with a VEGF receptor inhibitor shows clinical benefits. Vogelzang NJ et al., J Clin Oncol 27:15s, 2009 (suppl; abstract 5034) (2009 ASCO Annual Meeting); Cho DC et al., J Clin Oncol 27:15s, 2009 (suppl; abstract 5101) (2009 ASCO Annual Meeting). In some embodiments, the invention relates to treating renal cell carcinoma, comprising administering to the individual: a) an effective amount of a composition comprising nanoparticles comprising a taxane and a carrier protein (such as albumin); b) an effective amount of perifosine.

In some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of erlotinib. In some embodiments, the method of treating a proliferative disease (such as cancer) in an individual comprises administering to the individual: a) an effective amount of a composition comprising nanoparticles comprising paclitaxel coated with an albumin (such as Abraxane®); and b) an effective amount of erlotinib. In some embodiments, the nanoparticle composition and the erlotinib are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

Further, there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an inhibitor of PDK1.

Further, there is disclosed a method of treating a proliferative disease (such as cancer) in an individual, comprising administering to the individual a) an effective amount of a composition comprising nanoparticles comprising a taxane and a carrier protein (e.g., albumin) and b) an effective amount of at least one other agent that inhibits cyclin D1 (such as cycline D1 overexpression). The cancer may be mantle cell lymphoma and breast cancer.

Furhter, there is disclosed a method of treating a proliferative disease (such as cancer) in an individual, comprising administering to the individual a) an effective amount of a composition comprising nanoparticles comprising a taxane and a carrier protein (e.g., albumin) and b) an effective amount of at least one other agent that inhibits Myc over expression. The cancer may be burkitt lymphoma.

Further, there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that inhibits HIF. In some cases, the HIF is HIF1. In some cases, the HIF is HIF2. In some cases, the other agent inhibits HIF-mediated angiogenesis. In some embodiments, the other agent inhibitors HIF overexpression. The cancer may be RCC and/or Von Hippel-Lindau (VHL).

Other PI3K/Akt signaling pathway inhibitors that can be used in the methods described herein include, but are not limited to, e.g., FTY720 and UCN-01.

Further, there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the Mitogen-Activated Protein Kinase (MAP kinase) signaling pathway. The MAP kinase signaling pathway described herein include any members or components that directly or indirectly participate in the signal transduction cascade. These include, but are not limited to, MAP kinases such as Erk1, Erk2, p38 MAPK, SAPK, JNK, Erk5, BMK1; MAP kinase kinases such as MEK1, MEK2, MKK3, MKK6, MKK4, MKK7, MEK5; and MAP kinase kinase kinase such as Raf, Mos, Tp12, MLK3, TAK, DLK, MEKK1, MEKK4, MLK3, ASK1, MEKK2, and MEKK3. The agent that affects (such as inhibits) the MAP kinase pathway can thus act through modulation of any one or more of these components. There is disclosed a method of treating cancer in an individual, comprising administering to the individual: a) an effective amount of a composition comprising nanoparticles comprising a taxane and a carrier protein (such as albumin), and b) an effective amount of a compound that affects the MAPK pathway (e.g., sorafenib (BAY49-9006). The method may further comprise administering an effective amount of a tyrosine kinase inhibitor (e.g., geftinib or erlotinib). The proliferative disease may be non-small cell lung carcinoma. Alternatively, the proliferative disease may be brain cancer (e.g., glioblastoma).

In some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the bcl-2 signaling pathway. In some embodiments, the method of treating a proliferative disease (such as cancer) in an individual comprises administering to the individual: a) an effective amount of a composition comprising nanoparticles comprising paclitaxel coated with albumin (such as Abraxane®); and b) an effective amount of an agent that affects (such as inhibits) the bcl-2 signaling pathway. The bcl-2 signaling pathway described herein includes any members or components that directly or indirectly participate in the signal transduction cascade. These include, but are not limited to, JNK, Hrk, Bim, Bax, Noxa, and Puma. The agent that affects (such as inhibits) the bcl-2 signaling pathway can thus act through modulation of any one or more of these components.

Thus, for example, in some embodiments, the method of treating a proliferative disease (such as cancer) in an individual comprises administering to the individual: a) an effective amount of a composition comprising nanoparticles comprising a taxane and a carrier protein (such as albumin); and b) an effective amount of an agent that inhibits bcl-2. In some embodiments, the method of treating a proliferative disease (such as cancer) in an individual, comprising administering to the individual: a) an effective amount of a composition comprising nanoparticles comprising paclitaxel coated with albumin (such as Abraxane®); and b) an effective amount of an agent that inhibits bcl-2. In some embodiments, the other agent is a small molecule compound that inhibits the activity of bcl-2. In some embodiments, the small molecule compound is ABT-263, HA 14-1, Obatoclax, and ABT-737. In some embodiments, the agent is in an amount effective to suppress taxane-mediated upregulation of bcl-2 *in vivo.*

In some embodiments, the method of treating a proliferative disease (such as cancer) in an individual comprises administering to the individual: a) an effective amount of a composition comprising nanoparticles comprising a taxane (such as paclitaxel) and a carrier protein (such as albumin); and b) an effective amount of a small molecule inhibitor of bcl-2. In some embodiments, the method of treating a proliferative disease (such as cancer) in an individual comprises administering to the individual: a) an effective amount of a composition comprising nanoparticles comprising paclitaxel coated with albumin (such as Abraxane®); and b) an effective amount of a small molecule inhibitor of bcl-2. In some embodiments, the small molecule is in an amount effective to suppress taxane-mediated upregulation of bcl-2 *in vivo.* In some embodiments, the bcl-2 inhibitor is administered orally. In some embodiments, the nanoparticle composition and the bcl-2 inhibitor are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

In some embodiments, the method of treating a proliferative disease (such as cancer) in an individual comprises administering to the individual: a) an effective amount of a composition comprising nanoparticles comprising a taxane (such as paclitaxel) and a carrier protein (such as albumin), wherein the taxane is in the dosage range of about 60-300 mg/m²; and b) about 10-500 mg/day (including for example about 20-100, such as about 50 mg/dav) of a small molecule inhibitor of bcl-2. In some embodiments, the method of treating a proliferative disease (such as cancer) in an individual comprises administering to the individual: a) an effective amount of a composition comprising nanoparticles comprising paclitaxel coated with albumin (such as Abraxane®), wherein the taxane is in the dosage range of about 60-300 mg/m²; and b) about 10-500 mg/day (including for example about 20-100, such as about 50 mg/day) of a small molecule inhibitor of bcl-2. In some embodiments, the nanoparticle composition is administered intravenously. In some embodiments, the small molecule is in an amount effective to suppress taxane-mediated upregulation of bcl-2 *in vivo.* In some embodiments, the bcl-2 inhibitor is administered orally. In some embodiments, the nanoparticle composition and the bcl-2 inhibitor are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

In some embodiments, the inhibitor of bcl-2 is an antisense oligonucleotide that inhibits the expression of bcl-2. In some embodiments, the antisense oligonucleotide is an antisense oligodeoxynucleotide. In some embodiments, the antisense oligonucleotide is an antisense oligodeoxyribonucleotide. In some embodiments, the other agent is oblimersen. For example, in some embodiments, the method of treating a proliferative disease (such as cancer, for example melanoma, e.g., advanced melanoma) in an individual compries administering to the individual a) an effective amount of a composition comprising nanoparticles comprising paclitaxel and an albumin (such as Abraxane®), and b) an antisense oligonucleotide that inhibits the expression of bcl-2. In some embodiments, the method of treating a proliferative disease (such as cancer, for example melanoma, e.g., advanced melanoma) in an individual compries administering to the individual: a) an effective amount of a composition comprising nanoparticles comprising paclitaxel coated with an albumin (such as Abraxane®), and b) oblimersen.

In some embodiments, the method further comprises administering to the individual an alkylating agent, such as temozolomide. Thus, for example, in some embodiments, the method of treating a proliferative disease (such as cancer, for example melanoma, e.g., advanced melanoma) in an individual comprises administering to the individual: a) an effective amount of a composition comprising nanoparticles comprising paclitaxel coated with an albumin (such as Abraxane®), b) an antisense oligonucleotide that inhibits the expression of bcl-2 (such as oblimersen), and c) an alkylating agent (such as temozolomide). In some embodiments, the method of treating melanoma (such as advanced melanoma) in an individual comprises administering to the individual: a) an effective amount of nanoparticles comprising paclitaxel coated with albumin (such as Abraxane®), b) an effective amount of oblimersen, and c) an effective amount of temozolomide. In some embodiments, the individual has normal LDH.

In some embodiments, the invention relates to treating a proliferative disease (such as cancer) in an individual, comprising administering to the individual: a) an effective amount of a composition comprising nanoparticles comprising a taxane and a carrier protein (such as albumin); and b) an effective amount of an agent that inhibits bcl-xL. In some embodiments, the method of treating a proliferative disease (such as cancer) in an individual, comprising administering to the individual: a) an effective amount of a composition comprising nanoparticles comprising paclitaxel coated with albumin (such as Abraxane®); and b) an effective amount of an agent that inhibits bcl-xL. In some embodiments, the other agent is a small molecule compound that inhibits the activity of bcl-xL. In some embodiments, the other agent is an inhibitor of both bcl-2 and bcl-xL. In some embodiments, the other agent is in an amount effective to suppress taxane-mediated upregulation of bcl-2 *in vivo.* In some embodiments, the other agent is in an amount effective to suppress taxane-mediated upregulation of both bcl-2 and bcl-xL *in vivo.* In some embodiments, the other agent is administered orally. In some embodiments, the nanoparticle composition and the other agent are administered concurrently. In some embodiments, the proliferative disease is cancer selected from the group consisting of breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

The other agent may be an inhibitor of bcl-w.

Further, there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the TNF-a signaling pathway. The TNF-a signaling pathway described herein include any members or components that directly or indirectly participate in the signal transduction cascade. These include, but are not limited to, DAPK, FADD, TRADD, RIP, F2, RAIDD, IKKs, NF-κB, and IκB. The agent that affects (such as inhibits) the TNF-a signaling pathway can thus act through modulation of any one or more of these components.

Further, there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the NF-κB signaling pathway. The NF-κB signaling pathway described herein include any members or components that directly or indirectly participate in the signal transduction cascade. These include, but are not limited to, PKCζ, GSKβ, MSK1, IKKs, NAK, RSK1, NAP1, PKAc, CKII, TAK, RIP, as well as components involved in the TNF-a signaling pathway as described above. The agent that affects (such as inhibits) the NF-κB signaling pathway can thus act through modulation of anv one or more of these components.

Further there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the IL-6 signaling pathway. The IL-6 signaling pathway described herein include any members or components that directly or indirectly participate in the signal transduction cascade. These include, but are not limited to, Gp130, Jak, Stat3, Stat, SHP2, SOCS3, Grb2, Shc, Ras, Raf, MEK, Erk, PI3K, Akt, mTOR (TORC1, TORC2), IKKs, IκB, and NF-κB. The agent that affects (such as inhibits) the IL-6 signaling pathway can thus act through modulation of any one or more of these components.

Further there is disclosed a method of treating a proliferative disease (such as cancer) in an individual, comprising administering to the individual: a) an effective amount of a composition comprising nanoparticles comprising a taxane and a carrier protein (such as albumin); and b) an effective amount of an agent that affects (such as inhibits) the IL-8 signaling pathway. The IL-8 signaling pathway described herein include any members or components that directly or indirectly participate in the signal transduction cascade. The agent that affects (such as inhibits) the IL-8 signaling pathway can thus act through modulation of any one or more of these components.

In some embodiments, the methods further comprise administration of one or more additional agent. In some embodiments, the additional agent is another agent that inhibits a prosurvival or proinflammatory signal, such as the agents described herein. In some embodiments, the additional agent is a chemotherapeutic agent, such as chemotherapeutic agents described in U.S. Patent Application No. 2006/0263434. In some embodiments, the additional agent is any one of dexamethasone, bortezomib, imatinib, sorafenib, gemcitabine, capecitabine, lenalidomide, sunitinib, paclitaxel, and docetaxel. For example, in some embodiments, the method of treating a proliferative disease comprises: a) an effective amount of a composition comprising nanoparticles comprising a taxane (such as paclitaxel) and a carrier protein (such as albumin), b) an effective amount of perifosine, and c) an effective amount of an additional agent selected from the group consisting of dexamethasone, bortezomib, imatinib, sorafenib, gemcitabine, capecitabine, lenalidomide, sunitinib, paclitaxel, and docetaxel. In some embodiments, the method of treating a proliferative disease comprises: a) an effective amount of a composition comprising nanoparticles comprising a taxane (such as paclitaxel) and a carrier protein (such as albumin), b) an effective amount of perifosine, and c) an effective amount of capecitabine. In some embodiments, the method of treating a proliferative disease comprises: a) an effective amount of a composition comprising nanoparticles comprising a taxane (such as paclitaxel) and a carrier protein (such as albumin), b) an effective amount of perifosine, and c) an effective amount of erlotinib. In some embodiments, the method of treating a proliferative disease comprising: a) an effective amount of a composition comprising nanoparticles comprises a taxane (such as paclitaxel) and a carrier protein (such as albumin), b) an effective amount of a bcl-2 inhibitor, and c) an effective amount of an additional agent selected from the group consisting of dexamethasone, bortezomib, imatinib, sorafenib, gemcitabine, capecitabine, lenalidomide, sunitinib, paclitaxel, and docetaxel. In some embodiments, the method of treating a proliferative disease comprises: a) an effective amount of a composition comprising nanoparticles comprising a taxane (such as paclitaxel) and a carrier protein (such as albumin), b) an effective amount of a bcl-xL inhibitor and c) an effective amount of an additional agent selected from the group consisting of dexamethasone, bortezomib, imatinib, sorafenib, gemcitabine, capecitabine, lenalidomide, sunitinib, paclitaxel, and docetaxel. In some embodiments, the method of treating a proliferative disease comprises: a) an effective amount of a composition comprising nanoparticles comprising a taxane (such as paclitaxel) and a carrier protein (such as albumin), b) an effective amount of perifosine, and c) an effective amount of perifosine or erlotinib.

The present application also provides pharmaceutical compositions comprising nanoparticles comprising a taxane and a carrier protein (such as albumin) for use in the treatment of a proliferative disease (such as cancer), wherein said use comprises simultaneous, sequential, and/or concurrent administration of an agent that inhibits a prosurvival and/or inflammatory signal. In some embodiments, the invention provides a pharmaceutical composition comprising an agent that inhibits a prosurvival and/or inflammatory signal for use in the treatment of a proliferative disease (such as cancer), wherein said use comprises simultaneous, sequential, and/or concurrent administration of a composition comprising nanoparticles comprising a taxane and a carrier protein (such as albumin). In some embodiments, the invention provides taxane-containing nanoparticle compositions and compositions comprising an agent that inhibits prosurvival and/or inflammatory signal for simultaneous, sequential, and/or concurrent use for treatment of a proliferative disease (such as cancer).

### Methods of treating proliferative diseases

The combination therapy methods described herein are useful for treating proliferative diseases. In some embodiments, the invention relates to inhibiting cell proliferation (such as tumor growth) in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the effective amounts of the taxane nanoparticle composition and the other agent synergistically inhibit cell proliferation (such as tumor cell growth). In some embodiments, at least about 10% (including for example at least about any of 20%, 30%, 40%, 60%, 70%, 80%, 90%, or 100%) cell proliferation is inhibited. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

In some embodiments, the invention relates to inhibiting tumor metastasis (such as metastasis of breast cancer, pulmonary metastasis or metastasis to the lymph node) in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the effective amounts of the taxane nanoparticle composition and the other agent synergistically inhibit tumor metastasis. In some embodiments, at least about 10% (including for example at least about any of 20%, 30%, 40%, 60%, 70%, 80%, 90%, or 100%) metastasis is inhibited. In some embodiments, method of inhibiting metastasis to lymph node is provided. In some embodiments, method of inhibiting metastasis to the lung is provided. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

In some embodiments, the invention relates to reducing (such as eradiating) pre-existing tumor metastasis (such as pulmonary metastasis or metastasis to the lymph node) in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the effective amounts of the taxane nanoparticle composition and the other agent synergistically reduces (such as eradicates) tumor metastasis. In some embodiments, at least about 10% (including for example at least about any of 20%, 30%, 40%, 60%, 70%, 80%, 90%, or 100%) metastasis is reduced. In some embodiments, reducing metastasis to lymph node is provided. In some embodiments, reducing metastasis to the lung is provided. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

In some embodiments, the invention relates to reducing incidence or burden of preexisting tumor metastasis (such as pulmonary metastasis or metastasis to the lymph node) in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered bv oral administration.

In some embodiments, the invention relates to reducing tumor size in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the effective amounts of the taxane nanoparticle composition and the other agent synergistically reduces tumor size. In some embodiments, the tumor size is reduced at least about 10% (including for example at least about any of 20%, 30%, 40%, 60%, 70%, 80%, 90%, or 100%). In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

In some embodiments, the invention relates to prolonging time to disease progression of a proliferative disease (such as cancer) in an individual, comprising administering to the individual: a) an effective amount of a composition comprising nanoparticles comprising taxane and a carrier protein, and b) an effective amount of at least one other agent that inhibits a prosurvival and/or inflammatory signal. In some embodiments, the method prolongs the time to disease progression by at least any of 1, 2, 3, 4, 5, 6, 7, 8, 9, 10, 11, or 12 weeks. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

In some embodiments, the invention relates to prolonging survival of an individual having a proliferative disease (such as cancer), comprising administering to the individual: a) an effective amount of a composition comprising nanoparticles comprising taxane and a carrier protein, and b) an effective amount of at least one other agent that inhibits a prosurvival and/or inflammatory signal. In some embodiments, the method prolongs the survival of the individual by at least any of 1, 2, 3, 4, 5, 6, 7, 8, 9, 10, 11, 12, 18, or 24 month. In some embodiments, the taxane is paclitaxel. In some embodiments, the other agent is an inhibitor of Akt (such as perifosine and erlotinib). In some embodiments, the other agent is an inhibitor of bcl-2, bcl-xL, or both. In some embodiments, the taxane in the nanoparticle in the composition is administered by intravenous administration. In some embodiments, the other agent is administered by intraperitoneal administration. In some embodiments, the other agent is administered by oral administration.

It is understood that any of the embodiments in this section apply to the embodiments provided in the section "methods of combination therapy." For example, in some embodiments, the invention relates to reducing (such as eradiating) pre-existing tumor metastasis (such as pulmonary metastasis or metastasis to the lymph node) in an individual, comprising administering to the individual: a) an effective amount of a composition comprising nanoparticles comprising taxane and a carrier protein, and b) an effective amount of a perifosine, wherein the nanoparticle composition and the perifosine are administered concurrently. In some embodiments, the a method of reducing (such as eradiating) pre-existing tumor metastasis (such as pulmonary metastasis or metastasis to the lymph node) in an individual comprises administering to the individual: a) an effective amount of nanoparticles comprising paclitaxel coated with albumin (such as Abraxane®), and b) an effective amount of a perifosine, wherein the nanoparticle composition and the perifosine are administered concurrently. In some embodiments, the method of reducing (such as eradiating) pre-existing tumor metastasis (such as pulmonary metastasis or metastasis to the lymph node) in an individual comprises administering to the individual: a) an effective amount of a composition comprising nanoparticles comprising taxane and a carrier protein, and b) an effective amount of a bcl-2 inhibitor, wherein the nanoparticle composition and the bcl-2 inhibitor are administered concurrently. In some embodiments, the method of reducing (such as eradiating) pre-existing tumor metastasis (such as pulmonary metastasis or metastasis to the lymph node) in an individual comprises administering to the individual: a) an effective amount of nanoparticles comprising paclitaxel coated with albumin (such as Abraxane®), and b) an effective amount of a bcl-2 inhibitor, wherein the nanoparticle composition and the bcl-2 are administered concurrently.

The methods described herein are useful for treating various diseases, including for example, breast cancer, lung cancer (such as small cell lung cancer and non-small cell lung cancer), renal cancer, bladder cancer, pancreatic cancer, melanoma, prostate cancer, brain cancer, colorectal cancer, leukemia, and multiple myeloma.

### Modes of administration

The composition comprising nanoparticles comprising taxane (also referred to as "nanoparticle composition") and the other agent can be administered simultaneously (i.e., simultaneous administration) and/or sequentially (i.e., sequential administration).

In some embodiments, the nanoparticle composition and the other agent (including the specific agents described herein) are administered simultaneously. The term "simultaneous administration," as used herein, means that the nanoparticle composition and the other agent are administered with a time separation of no more than about 15 minute(s), such as no more than about any of 10, 5, or 1 minutes. When the drugs are administered simultaneously, the drug in the nanoparticles and the other agent may be contained in the same composition (e.g., a composition comprising both the nanoparticles and the other agent) or in separate compositions (e.g., the nanoparticles are contained in one composition and the other agent is contained in another composition).

In some embodiments, the nanoparticle composition and the other agent are administered sequentially. The term "sequential administration" as used herein means that the drug in the nanoparticle composition and the other agent are administered with a time separation of more than about 15 minutes, such as more than about any of 20, 30, 40, 50, 60 or more minutes. Either the nanoparticle composition or the other agent may be administered first. The nanoparticle composition and the other agent are contained in separate compositions, which may be contained in the same or different packages.

In some embodiments, the administration of the nanoparticle composition and the other agent are concurrent, i.e., the administration period of the nanoparticle composition and that of the other agent overlap with each other. In some embodiments, the nanoparticle composition is administered for at least one cycle (for example, at least any of 2, 3, or 4 cycles) prior to the administration of the other agent. In some embodiments, the other agent is administered for at least any of one, two, three, or four weeks. In some embodiments, the administrations of the nanoparticle composition and the other agent are initiated at about the same time (for example, within any one of 1, 2, 3, 4, 5, 6, or 7 days). In some embodiments, the administrations of the nanoparticle composition and the other agent are terminated at about the same time (for example, within any one of 1, 2, 3, 4, 5, 6, or 7 days). In some embodiments, the administration of the other agent continues (for example for about any one of 1, 2, 3, 4, 5, 6, 7, 8, 9, 10, 11, or 12 months) after the termination of the administration of the nanoparticle composition. In some embodiments, the administration of the other agent is initiated after (for example after about any one of 1, 2, 3, 4, 5, 6, 7, 8, 9, 10, 11, or we months) the initiation of the administration of the nanoparticle composition. In some embodiments, the administrations of the nanoparticle composition and the other agent are initiated and terminated at about the same time. In some embodiments, the administrations of the nanoparticle composition and the other agent are initiated at about the same time and the administration of the other agent continues (for example for about any one of 1, 2, 3, 4, 5, 6, 7, 8, 9, 10, 11, or 12 months) after the termination of the administration of the nanoparticle composition. In some embodiments, the administration of the nanoparticle composition and the other agent stop at about the same time and the administration of the other agent is initiated after (for example after about any one of 1, 2, 3, 4, 5, 6, 7, 8, 9, 10, 11, or we months) the initiation of the administration of the nanoparticle composition.

In some embodiments, the administration of the nanoparticle composition and the other agent are non-concurrent. For example, in some embodiments, the administration of the nanoparticle composition is terminated before the other agent is administered. In some embodiments, the administration of the other agent is terminated before the nanoparticle composition is administered. The time period between these two non-concurrent administrations can range from about two to eight weeks, such as about four weeks.

The dosing frequency of the drug-containing nanoparticle composition and the other agent may be adjusted over the course of the treatment, based on the judgment of the administering physician. When administered separately, the drug-containing nanoparticle composition and the other agent can be administered at different dosing frequency or intervals. For example, the drug-containing nanoparticle composition can be administered weekly, while a other agent can be administered more or less frequently. In some embodiments, sustained continuous release formulation of the drug-containing nanoparticle and/or other agent may be used. Various formulations and devices for achieving sustained release are known in the art. Exemplary dosing frequencies are further provided herein.

The nanoparticle composition and the other agent can be administered using the same route of administration or different routes of administration. Exemplary administration routes are further provided herein. In some embodiments (for both simultaneous and sequential administrations), the taxane in the nanoparticle composition and the other agent are administered at a predetermined ratio. For example, in some embodiments, the ratio by weight of the taxane in the nanoparticle composition and the other agent is about 1 to 1. In some embodiments, the weight ratio may be between about 0.001 to about 1 and about 1000 to about 1, or between about 0.01 to about 1 and 100 to about 1. In some embodiments, the ratio by weight of the taxane in the nanoparticle composition and the other agent is less than about any of 100:1, 50:1, 30:1, 10:1, 9:1, 8:1, 7:1, 6:1, 5:1, 4:1, 3:1, 2:1, and 1:1 In some embodiments, the ratio by weight of the taxane in the nanoparticle composition and the other agent is more than about any of 1:1, 2:1, 3:1, 4:1, 5:1, 6:1, 7:1, 8:1, 9:1, 30:1, 50:1, 100:1. Other ratios are contemplated.

The methods described herein comprise administration of taxane and the other agent in effective amounts. In some embodiments, there is provided a method of treating a proliferative disease (such as cancer, for example breast cancer) in an individual, comprising administering to the individual: a) an effective amount of a composition comprising taxane, and b) an effective amount of an the other agent (such as an Akt inhibitor, for example perifosine), wherein the other agent is an amount effective to suppress taxane-mediated upregulation of the prosurvival and/or inflammatory signal (such as Akt) *in vivo.* In some embodiments, there is provided a method of inhibiting tumor metastasis (such as breast cancer metastasis) in an individual, comprising administering to the individual: a) an effective amount of a composition comprising taxane, and b) an effective amount of an the other agent (such as an Akt inhibitor, for example perifosine), wherein the other agent is an amount effective to suppress taxane-mediated upregulation of the prosurvival and/or inflammatory signal (such as Akt) *in vivo.* In some embodiments, the taxane-mediated upregulation of the prosurvival and/or inflammatory signal *in vivo* is taxane-mediated upregulation of Akt. In some embodiments, the taxane-mediated upregulation of the prosurvival and/or inflammatory signal *in vivo* is taxane-mediated upregulation of any of bcl-2, bcl-xL, IL-6, IL-8, TNF-a, as well as phosphorylated NF-κB p65, p50, and p42/44 kinase. In some embodiments, the amount of the other agent is effective to abrogate or reduce stress response elicited by taxane. In some embodiments, the taxane is paclitaxel. In some embodiments, the taxane is docetaxel. In some embodiments, the composition comprising a taxane is a composition comprising nanoparticles comprising a taxane and a carrier protein. In some embodiments, the nanoparticles comprising a taxane are nanoparticles comprising paclitaxel. In some embodiments, the nanoparticles comprising a taxane are nanoparticles comprising docetaxel.

The term "amount effective to suppress taxane-mediated upregulation" of a prosurvival and/or inflammatory signal, as used herein, refers to and includes both complete (including substantially complete) and/or partial suppression. Methods indicating such suppression are known in the art and described herein, although it is understood that when administering to an individual patient based on established medical practice on the basis of clinical trials, such measurements need not be given in an individual. In some embodiments, the term "amount effective to suppress taxane-mediated upregulation of Akt," as used herein, refers to substantially complete prevention of Akt expression and/or activity or reduction in the amount of Akt expression and/or activity in cells, tissues or fluids *in vivo* upon administration of a formulation containing a taxane. In some embodiments, the reduction in the amount Akt expression and/or activity in cells, tissues or fluids *in vivo* upon administration of a formulation containing a taxane is by at least about any of 10%, 20%, 30%, 40%, 50%, 60%, 70%, 80%, 90%, 95%, or 100%. In some embodiments, the suppression of taxane induction can be observed qualitatively and/or quantitatively by methods known in the art and described herein.

The doses required for the taxane and/or the other agent may (but not necessarily) be lower than what is normally required when each agent is administered alone. Thus, in some embodiments, a subtherapeutic amount of the drug in the nanoparticle composition and/or the other agent are administered. "Subtherapeutic amount" or "subtherapeutic level" refer to an amount that is less than therapeutic amount, that is, less than the amount normally used when the drug in the nanoparticle composition and/or the other agent are administered alone. The reduction may be reflected in terms of the amount administered at a given administration and/or the amount administered over a given period of time (reduced frequency).

In some embodiments, enough other agent is administered so as to allow reduction of the normal dose of the drug in the nanoparticle composition required to effect the same degree of treatment by at least about any of 5%, 10%, 20%, 30%, 50%, 60%, 70%, 80%, 90%, or more. In some embodiments, enough drug in the nanoparticle composition is administered so as to allow reduction of the normal dose of the other agent required to effect the same degree of treatment by at least about any of 5%, 10%, 20%, 30%, 50%, 60%, 70%, 80%, 90%, or more.

In some embodiments, the dose of both the taxane in the nanoparticle composition and the other agent are reduced as compared to the corresponding normal dose of each when administered alone. In some embodiments, both the taxane in the nanoparticle composition and the other agent are administered at a subtherapeutic, i.e., reduced, level. In some embodiments, the dose of the nanoparticle composition and/or the other agent is substantially less than the established maximum toxic dose (MTD). For example, the dose of the nanoparticle composition and/or the other agent is less than about 50%, 40%, 30%, 20%, or 10% of the MTD.

In some embodiments, the dose of taxane and/or the dose of the other agent is higher than what is normally required when each agent is administered alone. For example, in some embodiments, the dose of the nanoparticle composition and/or the other agent is substantially higher than the established maximum toxic dose (MTD). For example, the dose of the nanoparticle composition and/or the other agent is more than about 50%, 40%, 30%, 20%, or 10% of the MTD of the agent when administered alone.

In some embodiments, the amount of a taxane (e.g., paclitaxel) in the composition is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of a taxane (e.g., paclitaxel) or derivative thereof in the effective amount of the composition (e.g., a unit dosage form) is in the range of about 5 mg to about 500 mg, such as about 30 mg to about 300 mg or about 50 mg to about 200 mg. In some embodiments, the concentration of the taxane (e.g., paclitaxel) in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the taxane (e.g., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (e.g., paclitaxel) in the nanoparticle composition include, but are not limited to, at least about any of 25 mg/m², 30 mg/m², 50 mg/m², 60 mg/m², 75 mg/m², 80 mg/m², 90 mg/m², 100 mg/m², 120 mg/m², 125 mg/m², 150 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of a taxane (e.g., paclitaxel). In various embodiments, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of a taxane (e.g., paclitaxel). In some embodiments, the amount of the taxane (e.g., paclitaxel) per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of a taxane (e.g., paclitaxel) in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². In some embodiments, the effective amount of a taxane (e.g., paclitaxel) in the composition is about 5 to about 300 mg/m², such as about 20 to about 300 mg/m², about 50 to about 250 mg/m², about 100 to about 150 mg/m², about 120 mg/m², about 130 mg/m², or about 140 mg/m², or about 260 mg/m².

In some embodiments of any of the above aspects, the effective amount of a taxane (e.g., paclitaxel) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various embodiments, the effective amount of a taxane (e.g., paclitaxel) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (e.g., paclitaxel).

Exemplary dosing frequencies for the nanoparticle composition (and as indicated below for the other agent) include, but are not limited to, weekly without break; weekly, three out of four weeks; once every three weeks; once every two weeks; weekly, two out of three weeks. In some embodiments, the composition is administered about once every 2 weeks, once every 3 weeks, once every 4 weeks, once every 6 weeks, or once every 8 weeks. In some embodiments, the composition is administered at least about any of 1x, 2x, 3x, 4x, 5x, 6x, or 7x (i.e., daily) a week, or three times daily, two times daily. In some embodiments, the intervals between each administration are less than about any of 6 months, 3 months, 1 month, 20 days, 15 days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

In some embodiments, the taxane in the nanoparticle composition is administered weekly. In some embodiments, the taxane in a nanoparticle composition is administered every two weeks. In some embodiments, the taxane in the nanoparticle composition is administered every three weeks. In some embodiments, the other agent is administered 1x, 2x, 3x, 4x, 5x, 6x, or 7 times a week. In some embodiments, the other agent is administered every two weeks or two out of three weeks. In some embodiments, the taxane is paclitaxel. In some embodiment, the other agent is perifosine. In some embodiments of the above dosages and/or administrations, the taxane is paclitaxel and the other agent is perifosine.

The administration of the nanoparticle composition (and for the other agent) can be extended over an extended period of time, such as from about a month up to about seven years. In some embodiments, the composition is administered over a period of at least about any of 2, 3, 4, 5, 6, 7, 8, 9, 10, 11, 12, 18, 24, 30, 36, 48, 60, 72, or 84 months. In some embodiments, the taxane (e.g., paclitaxel) is administered over a period of at least one month, wherein the interval between each administration is no more than about a week, and wherein the dose of the taxane (e.g., paclitaxel) at each administration is about 0.25 mg/m² to about 75 mg/m², such as about 0.25 mg/m² to about 25 mg/m² or about 25 mg/m² to about 50 mg/m².

In some embodiments, the dosage of a taxane (e.g., paclitaxel) in a nanoparticle composition can be in the range of 5-400 mg/m² when given on a 3 week schedule, or 5-250 mg/m² when given on a weekly schedule. For example, the amount of a taxane (e.g., paclitaxel) can be about 60 to about 300 mg/m² (e.g., about 260 mg/m²) when given on a three week schedule.

Other exemplary dosing schedules for the administration of the nanoparticle composition (e.g., paclitaxel/albumin nanoparticle composition) include, but are not limited to, 100 mg/m², weekly, without break; 75 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m², twice a week; and 150-250 mg/m² twice a week. The dosing frequency of the composition may be adjusted over the course of the treatment based on the judgment of the administering physician.

In some embodiments, the individual is treated for at least about any of one, two, three, four, five, six, seven, eight, nine, or ten treatment cycles. The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

Other exemplary dose of the taxane (in some embodiments paclitaxel) in the nanoparticle composition include, but is not limited to, about any of 50 mg/m², 60 mg/m², 75 mg/m², 80 mg/m², 90 mg/m², 100 mg/m², 120 mg/m², 160 mg/m², 175 mg/m², 200 mg/m², 210 mg/m², 220 mg/m², 260 mg/m², and 300 mg/m². For example, the dosage of paclitaxel in a nanoparticle composition can be in the range of about 100-400 mg/m² when given on a 3 week schedule, or about 50-250 mg/m² when given on a weekly schedule.

The dosing frequency of the other agent can be the same or different from that of the nanoparticle composition. Exemplary frequencies are provided above. As further example, the other agent can be administered three times a day, two times a day, daily, 6 times a week, 5 times a week, 4 times a week, 3 times a week, two times a week, weekly.. In some embodiments, the other agent is administered twice daily or three times daily. Exemplary amounts of the other agent include, but are not limited to, any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. For example, the other agent can be administered at a dose of about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). For example, in some embodiments, perifosine is administered (for example by oral administration) at about 20-100 mg/kg (including for example 30 mg/kg, 40 mg/kg, 50 mg/kg, 60 mg/kg, 70 mg/kg, 80 mg/kg), three times a week. In some embodiments, erlotinib is administered (for example by intraperitoneal administration) at about 20-200 mg/kg/day (including for example 50 mg/kg/day, 80 mg/kg/day, 100 mg/kg/day, 120 mg/kg/day, 140 mg/kg/day, 180 mg/kg/day).

In some embodiments, the effective amount of taxane in the nanoparticle composition is between about 45 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 300 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 150 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 80 mg/m² to about 150 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle composition is about 100 mg/m². In some embodiments, the effective amount of taxane in the nanoparticle composition is between about 170 mg/m² to about 200 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle composition is between about 200 mg/m² to about 350 mg/m² and the effective amount of the other agent is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle composition is about 260 mg/m². In some embodiments of any of the above methods, the effective amount of the other agent is about 20-30 mg/kg, about 30-40 mg/kg, about 40-50 mg/kg, about 50-60 mg/kg, about 60-70 mg/kg, about 70-80 mg/kg, about 80-100 mg/kg, or about 100-120 mg/kg.

The nanoparticle composition (and the other agent) described herein can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In one variation of the invention, nanoparticles (such as albumin nanoparticles) of the inventive compounds can be administered by any acceptable route including, but not limited to, orally, intramuscularly, transdermally, intravenously, through an inhaler or other air borne delivery systems and the like.

A combination of the administration configurations described herein can be used. The combination therapy methods described herein may be performed alone or in conjunction with another therapy, such as surgery, radiation, chemotherapy, immunotherapy, gene therapy, and the like. Additionally, a person having a greater risk of developing the proliferative disease may receive treatments to inhibit or and/or delay the development of the disease.

As will be understood by those of ordinary skill in the art, the appropriate doses of other agents will be approximately those already employed in clinical therapies wherein the other agent are administered alone or in combination with other agents. Variation in dosage will likely occur depending on the condition being treated. As described above, in some embodiments, the other agents may be administered at a reduced level.

### Proliferative diseases

The methods described herein are useful for treating proliferative diseases. In some embodiments, the proliferative disease is cancer.

Examples of cancers that may be treated by the methods of the invention include, but are not limited to, adenocortical carcinoma, agnogenic myeloid metaplasia, AIDS-related cancers (e.g., AIDS-related lymphoma), anal cancer, appendix cancer, astrocytoma (e.g., cerebellar and cerebral), basal cell carcinoma, bile duct cancer (e.g., extrahepatic), bladder cancer, bone cancer, (osteosarcoma and malignant fibrous histiocytoma), brain tumor (e.g., glioma, brain stem glioma, cerebellar or cerebral astrocytoma (e.g., pilocytic astrocytoma, diffuse astrocytoma, anaplastic (malignant) astrocytoma), malignant glioma, ependymoma, oligodenglioma, meningioma, craniopharyngioma, haemangioblastomas, medulloblastoma, supratentorial primitive neuroectodermal tumors, visual pathway and hypothalamic glioma, and glioblastoma), breast cancer, bronchial adenomas/carcinoids, carcinoid tumor (e.g., gastrointestinal carcinoid tumor), carcinoma of unknown primary, central nervous system lymphoma, cervical cancer, colon cancer, colorectal cancer, chronic myeloproliferative disorders, endometrial cancer (e.g., uterine cancer), ependymoma, esophageal cancer, Ewing's family of tumors, eye cancer (e.g., intraocular melanoma and retinoblastoma), gallbladder cancer, gastric (stomach) cancer, gastrointestinal carcinoid tumor, gastrointestinal stromal tumor (GIST), germ cell tumor, (e.g., extracranial, extragonadal, ovarian), gestational trophoblastic tumor, head and neck cancer, hepatocellular (liver) cancer (e.g., hepatic carcinoma and heptoma), hypopharyngeal cancer, islet cell carcinoma (endocrine pancreas), laryngeal cancer, laryngeal cancer, leukemia, lip and oral cavity cancer, oral cancer, liver cancer, lung cancer (e.g., small cell lung cancer, non-small cell lung cancer, adenocarcinoma of the lung, and squamous carcinoma of the lung), lymphoid neoplasm (e.g., lymphoma), medulloblastoma, melanoma, mesothelioma, metastatic squamous neck cancer, mouth cancer, multiple endocrine neoplasia syndrome, myelodysplastic syndromes, myelodysplastic/myeloproliferative diseases, nasal cavity and paranasal sinus cancer, nasopharyngeal cancer, neuroblastoma, neuroendocrine cancer, oropharyngeal cancer, ovarian cancer (e.g., ovarian epithelial cancer, ovarian germ cell tumor, ovarian low malignant potential tumor), pancreatic cancer, parathyroid cancer, penile cancer, cancer of the peritoneal, pharyngeal cancer, pheochromocytoma, pineoblastoma and supratentorial primitive neuroectodermal tumors, pituitary tumor, pleuropulmonary blastoma, lymphoma, primary central nervous system lymphoma (microglioma), pulmonary lymphangiomyomatosis, rectal cancer, renal cancer, renal pelvis and ureter cancer (transitional cell cancer), rhabdomyosarcoma, salivary gland cancer, skin cancer (e.g., non-melanoma (e.g., squamous cell carcinoma), melanoma, and Merkel cell carcinoma), small intestine cancer, squamous cell cancer, testicular cancer, throat cancer, thymoma and thymic carcinoma, thyroid cancer, tuberous sclerosis, urethral cancer, vaginal cancer, vulvar cancer, Wilms' tumor, and post-transplant lymphoproliferative disorder (PTLD), abnormal vascular proliferation associated with phakomatoses, edema (such as that associated with brain tumors), and Meigs' syndrome.

In some embodiments, the cancer is a solid tumor (such as advanced solid tumor). Solid tumor includes, but is not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine sacronomasynovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma (including for example adenocarcinoma, clear cell renal cell carcinoma, papillary renal cell carcinoma, chromophobe renal cell carcinoma, collecting duct renal cell carcinoma, granular renal cell carcinoma, mixed granular renal cell carcinoma, renal angiomyolipomas, or spindle renal cell carcinoma.), hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

In some embodiments the lymphoid neoplasm (*e.g*., lymphoma) is a B-cell neoplasm. Examples of B-cell neoplasms include, but are not limited to, precursor B-cell neoplasms (*e.g*., precursor B-lymphoblastic leukemia/lymphoma) and peripheral B-cell neoplasms (*e.g*., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (*e.g*., cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (*e.g*., extranodal (*e.g.*, MALT-type +/- monocytoid B cells) and/or Nodal (*e.g.*, +/monocytoid B cells)), splenic marginal zone lymphoma (*e.g*., +/- villous lymphocytes), Hairy cell leukemia, plasmacytoma/plasma cell myeloma (*e.g*., myeloma and multiple myeloma), diffuse large B-cell lymphoma (*e.g*., primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm (*e.g*., lymphoma) is a T-cell and/or putative NK-cell neoplasm. Examples of T-cell and/or putative NK-cell neoplasms include, but are not limited to, precursor T-cell neoplasm (precursor T-lymphoblastic lymphoma/leukemia) and peripheral T-cell and NK-cell neoplasms (*e.g.*, T-cell chronic lymphocytic leukemia/prolymphocytic leukemia, and large granular lymphocyte leukemia (LGL) (*e.g.*, T-cell type and/or NK-cell type), cutaneous T-cell lymphoma (*e.g*., mycosis fungoides/Sezary syndrome), primary T-cell lymphomas unspecified (*e.g*., cytological categories (*e.g.*, medium-sized cell, mixed medium and large cell), large cell, lymphoepitheloid cell, subtype hepatosplenic γd T-cell lymphoma, and subcutaneous panniculitic T-cell lymphoma), angioimmunoblastic T-cell lymphoma (AILD), angiocentric lymphoma, intestinal T-cell lymphoma (*e.g*., +/- enteropathy associated), adult T-cell lymphoma/leukemia (ATL), anaplastic large cell lymphoma (ALCL) (*e.g.*, CD30+, T- and null-cell types), anaplastic large-cell lymphoma, and Hodgkin's like).

In some embodiments the lymphoid neoplasm (*e.g*., lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, the cancer is leukemia. In some embodiments, the leukemia is chronic leukemia. Examples of chronic leukemia include, but are not limited to, chronic myelocytic I (granulocytic) leukemia, chronic myelogenous, and chronic lymphocytic leukemia (CLL). In some embodiments, the leukemia is acute leukemia. Examples of acute leukemia include, but are not limited to, acute lymphoblastic leukemia (ALL), acute myeloid leukemia, acute lymphocytic leukemia, and acute myelocytic leukemia (e.g., myeloblastic, promyelocytic, myelomonocytic, monocytic, and erythroleukemia).

In some embodiments, the cancer is liquid tumor or plasmacytoma. Plasmacytoma includes, but is not limited to, myeloma. Myeloma includes, but is not limited to, an extramedullary plasmacytoma, a solitary myeloma, and multiple myeloma. In some embodiments, the plasmacytoma is multiple myeloma.

In some embodiments, the cancer is multiple myeloma. Examples of multiple myeloma include, but are not limited to, IgG multiple myeloma, IgA multiple myeloma, IgD multiple myeloma, IgE multiple myeloma, and nonsecretory multiple myeloma. In some embodiments, the multiple myeloma is IgG multiple myeloma. In some embodiments, the multiple myeloma is IgA multiple myeloma. In some embodiments, the multiple myeloma is a smoldering or indolent multiple myeloma. In some embodiments, the multiple myeloma is progressive multiple myeloma. In some embodiments, multiple myeloma may be resistant to a drug, such as, but not limited to, bortezomib, dexamethasone (Dex-), doxorubicin (Dox-), and melphalan (LR).

In some embodiments, the cancer is selected from the group consisting of phosphorylated-Akt positive advanced solid tumors, non-small cell lung cancer, sarcoma, Waldenstrom's macroglobulinemia, malignant melanoma, sarcoma, refractory and relapsed leukemia, Androgen-independent prostate cancer, advanced pancreatic cancer, recurrent, hormone sensitive prostate cancer, metastatic HNSCC, metastatic breast cancer, multiple myeloma, colorectal cancer, ovarian cancer, head and neck cancer, GIST, and relapsed epithelial ovarian cancer.

### Nanoparticle compositions

The nanoparticle compositions described herein comprise nanoparticles comprising (in various embodiments consisting essentially of) a taxane (such as paclitaxel) and a carrier protein (such as albumin). Nanoparticles of poorly water soluble drugs (such as taxane) have been disclosed in, for example, U.S. Pat. Nos. 5,916,596; 6,506,405; 6,749,868, and 6,537,579 and also in U.S. Pat. Pub. Nos. 2005/0004002, 2006/0263434, and 2007/0082838; PCT Patent Application WO08/137148.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 to about 400 nm. In some embodiments, the average or mean diameter of the nanoparticles is about 40 to about 200 nm. In some embodiments, the nanoparticles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 400 nm, including for example about 20 to about 200 nm, abiyt 40 to about 200 nm, about 30 to about 180 nm, and any one of about 40 to about 150, about 50 to about 120, and about 60 to about 100 nm.

In some embodiments, the carrier protein has sulfhydral groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane(such as paclitaxel) coated with a carrier protein, such as albumin (e.g., human serum albumin). In some embodiments, the composition comprises taxane in both nanoparticle and non-nanoparticle forms, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the nanoparticle composition is substantially free (such as free) of surfactants (such as Cremophor®, Tween 80, or other organic solvents used for the administration of taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and taxane in the nanoparticle composition is about 18:1 or less, such as about 15:1 or less, for example about 10:1 or less. In some embodiments, the weight ratio of carrier protein (such as albumin) and taxane in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and taxane in the nanoparticle portion of the composition is about any one of 1:2, 1:3, 1:4, 1:5, 1:10, 1:15, or less. In some embodiments, the weight ratio of the carrier protein (such as albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

The nanoparticles described herein comprise a taxane and a carrier protein. The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art. The proteins described herein may be naturally occurring, i.e., obtained or derived from a natural source (such as blood), or synthesized (such as chemically synthesized or by synthesized by recombinant DNA techniques).

Examples of suitable carrier proteins include proteins normally found in blood or plasma, which include, but are not limited to, albumin, immunoglobulin including IgA, lipoproteins, apolipoprotein B, alpha-acid glycoprotein, beta-2-macroglobulin, thyroglobulin, transferin, fibronectin, factor VII, factor VIII, factor IX, factor X, and the like. In some embodiments, the carrier protein is non-blood protein, such as casein, a-lactalbumin, and β-lactoglobulin. The carrier proteins may either be natural in origin or synthetically prepared. In some embodiments, the pharmaceutically acceptable carrier comprises albumin, such as human serum albumin. Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolumic shock (see, e.g., Tullis, JAMA, 237, 355-360, 460-463, (1977)) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context).

Human serum albumin (HSA) has multiple hydrophobic binding sites (a total of eight for fatty acids, an endogenous ligand of HSA) and binds a diverse set of taxanes, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (198a), Vorum, Dan. Med. Bull., 46, 379-99 (1999), Kragh-Hansen, Dan. Med. Bull., 1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Adv. Protein. Chem., 45, 153-203 (1994)). Paclitaxel and propofol have been shown to bind HSA (see, e.g., Paal et al., Eur. J. Biochem., 268(7), 2187-91 (200a), Purcell et al., Biochim. Biophys. Acta, 1478(a), 61-8 (2000), Altmayer et al., Arzneimittelforschung, 45, 1053-6 (1995), and Garrido et al., Rev. Esp. Anestestiol. Reanim., 41, 308-12 (1994)). In addition, docetaxel has been shown to bind to human plasma proteins (see, e.g., Urien et al., Invest. New Drugs, 14(b), 147-51 (1996)).

The carrier protein (such as albumin) in the composition generally serves as a carrier for the taxane, i.e., the carrier protein in the composition makes the taxane more readily suspendable in an aqueous medium or helps maintain the suspension as compared to compositions not comprising a carrier protein. This can avoid the use of toxic solvents (or surfactants) for solubilizing the taxane, and thereby can reduce one or more side effects of administration of the taxane into an individual (such as a human). Thus, in some embodiments, the composition described herein is substantially free (such as free) of surfactants, such as Cremophor (including Cremophor EL® (BASF)). In some embodiments, the nanoparticle composition is substantially free (such as free) of surfactants. A composition is "substantially free of Cremophor" or "substantially free of surfactant" if the amount of Cremophor or surfactant in the composition is not sufficient to cause one or more side effect(s) in an individual when the nanoparticle composition is administered to the individual.

The amount of carrier protein in the composition described herein will vary depending on other components in the composition. In some embodiments, the composition comprises a carrier protein in an amount that is sufficient to stabilize the taxane in an aqueous suspension, for example, in the form of a stable colloidal suspension (such as a stable suspension of nanoparticles). In some embodiments, the carrier protein is in an amount that reduces the sedimentation rate of the taxane in an aqueous medium. For particle-containing compositions, the amount of the carrier protein also depends on the size and density of nanoparticles of the taxane.

A taxane is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4 °C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40 °C.

In some embodiments, the carrier protein is present in an amount that is sufficient to stabilize the taxane in an aqueous suspension at a certain concentration. For example, the concentration of the taxane in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg /ml. In some embodiments, the concentration of the taxane is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the carrier protein is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (e.g. about 0.5% (w/v), about 5% (w/v), about 10% (w/v), about 15% (w/v), about 20% (w/v), about 30% (w/v), about 40% (w/v), or about 50% (w/v)) of carrier protein. In some embodiments, the composition, in liquid form, comprises about 0.5% to about 5% (w/v) of carrier protein.

In some embodiments, the weight ratio of carrier protein, e.g., albumin, to the taxane in the nanoparticle composition is such that a sufficient amount of taxane binds to, or is transported by, the cell. While the weight ratio of carrier protein to taxane will have to be optimized for different carrier protein and taxane combinations, generally the weight ratio of carrier protein, e.g., albumin, to taxane (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, or about 9:1. In some embodiments, the carrier protein to taxane weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less. In some embodiments, the weight ratio of the carrier protein (such as albumin) and the taxane in the composition is any one of the following: about 1:1 to about 18:1, about 1:1 to about 15:1, about 1:1 to about 12:1, about 1:1 to about 10:1, about 1:1 to about 9:1, about 1:1 to about 8:1, about 1:1 to about 7:1, about 1:1 to about 6:1, about 1:1 to about 5:1, about 1:1 to about 4:1, about 1:1 to about 3:1, about 1:1 to about 2:1, about 1:1 to about 1:1.

In some embodiments, the carrier protein allows the composition to be administered to an individual (such as human) without significant side effects. In some embodiments, the carrier protein (such as albumin) is in an amount that is effective to reduce one or more side effects of administration of the taxane to a human. The term "reducing one or more side effects of administration of the taxane" refers to reduction, alleviation, elimination, or avoidance of one or more undesirable effects caused by the taxane, as well as side effects caused by delivery vehicles (such as solvents that render the taxanes suitable for injection) used to deliver the taxane. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with taxanes can be reduced.

In some embodiments, the composition comprises Abraxane® (*nab-*paclitaxel). Abraxane® is a formulation of paclitaxel stabilized by human albumin USP, which can be dispersed in directly injectable physiological solution. When dispersed in a suitable aqueous medium such as 0.9% sodium chloride injection or 5% dextrose injection, Abraxane® forms a stable colloidal suspension of paclitaxel. The mean particle size of the nanoparticles in the colloidal suspension is about 130 nanometers. Since HSA is freely soluble in water, Abraxane® can be reconstituted in a wide range of concentrations ranging from dilute (0.1 mg/ml paclitaxel) to concentrated (20 mg/ml paclitaxel), including for example about 2 mg/ml to about 8 mg/ml, about 5 mg/ml.

Methods of making nanoparticle compositions are known in the art. For example, nanoparticles containing taxanes (such as paclitaxel) and carrier protein (such as albumin) can be prepared under conditions of high shear forces (e.g., sonication, high pressure homogenization, or the like). These methods are disclosed in, for example, U.S. Pat. Nos. 5,916,596; 6,506,405; 6,749,868, and 6,537,579 and also in U.S. Pat. Pub. No. 2005/0004002, 2007/0082838, 2006/0263434and PCT Application WO08/137148.

Briefly, the taxane (such as paclitaxel) is dissolved in an organic solvent, and the solution can be added to a human serum albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/ethanol (for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1.

### Other components in the nanoparticle compositions

The nanoparticles described herein can be present in a composition that include other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines: palmitoyloleoylphosphatidylcholine, palmitoyllinoleoylphosphatidylcholine, stearoyllinoleoylphosphatidylcholine stearoyloleoylphosphatidylcholine, stearoylarachidoylphosphatidylcholine, and dipalmitoylphosphatidylcholine. Other phospholipids including L-a-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, e.g., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, e.g., U.S. Pat. Nos. 5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Kits, medicines, and compositions

The application also discloses kits, medicines, and compositions for use in the instant methods.

Kits include one or more containers comprising taxane-containing nanoparticle compositions (or unit dosage forms and/or articles of manufacture) and/or at least one other agent that inhibits a prosurvival and/or inflammatory signal, and in some embodiments, further comprise instructions for use in accordance with any of the methods described herein. The kit may further comprise a description of selection an individual suitable or treatment. Instructions supplied in the kits of the invention are typically written instructions on a label or package insert (e.g., a paper sheet included in the kit), but machine-readable instructions (e.g., instructions carried on a magnetic or optical storage disk) are also acceptable.

In some cases, the kit comprises a) a composition comprising nanoparticles comprising a taxane and a carrier protein (such as albumin), and b) an effective amount of at least one other agent that inhibits a prosurvival and/or inflammatory signal. In some cases, the kit comprises a) a composition comprising nanoparticles comprising a taxane and a carrier protein (such as albumin), b) an effective amount of at least one other agent that inhibits a prosurvival and/or inflammatory signal, and c) instructions for administering the nanoparticles and the other agents simultaneously, sequentially, or concurrently for treatment of a proliferative disease (such as cancer). In some cases, the taxane is any of paclitaxel, docetaxel, and ortataxel. In some cases, the kit comprises nanoparticles comprising a) a composition comprising nanoparticles comprising paclitaxel coated with an albumin (such as Abraxane®), b) an effective amount of at least one other agent that inhibits a prosurvival and/or inflammatory signal, and c) instructions for administering the nanoparticles and the other agents simultaneously, sequentially, and/or concurrently, for the effective treatment of a proliferative disease (such as cancer).

In some cases, the kit comprises a) a composition comprising nanoparticles comprising a taxane coated with a carrier protein (such as albumin), b) a composition comprising nanoparticles comprising at least one other agent that inhibits a prosurvival and/or inflammatory signal and a carrier protein (such as albumin), and c) instructions for administering the nanoparticle compositions simultaneously, sequentially, and/or concurrently, for treatment of a proliferative disease (such as cancer). In some cases, the kit comprises nanoparticles comprising a) a composition comprising nanoparticles comprising paclitaxel coated with an albumin (such as Abraxane®), b) a composition comprising nanoparticles comprising at least one other agent that inhibits a prosurvival and/or inflammatory signal and a carrier protein (such as albumin), and c) instructions for administering the nanoparticle compositions simultaneously, sequentially, and/or concurrently, for the effective treatment of a proliferative disease (such as cancer).

The nanoparticles and the other agents can be present in separate containers or in a single container. It is understood that the kit may comprise one distinct composition or two or more compositions wherein one composition comprises nanoparticles and one composition comprises an other agent.

The kits are in suitable packaging. Suitable packaging include, but is not limited to, vials, bottles, jars, flexible packaging (e.g., seled Mylar or plastic bags), and the like. Kits may optionally provide additional components such as buffers and interpretative information. The present application thus also discloses articles of manufacture, which include vials (such as sealed vials), bottles, jars, flexible packaging, and the like.

The instructions relating to the use of the nanoparticle compositions generally include information as to dosage, dosing schedule, and route of administration for the intended treatment. The containers may be unit doses, bulk packages (e.g., multi-dose packages) or sub-unit doses. For example, kits may be provided that contain sufficient dosages of the taxane (such as taxane) as disclosed herein to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the taxane and pharmaceutical compositions and instructions for use and packaged in quantities sufficient for storage and use in pharmacies, for example, hospital pharmacies and compounding pharmacies.

Also discloses are medicines for treating proliferative diseases. In some cases, the medicine comprises a) a composition comprising nanoparticles comprising a taxane and a carrier protein (such as albumin), and b) at least one other agent that inhibits a prosurvival and/or inflammatory signal. In some embodiments, the taxane is any of paclitaxel, docetaxel, and ortataxel. In some cases, the kit comprises nanoparticles comprising a) a composition comprising nanoparticles comprising paclitaxel coated with an albumin (such as Abraxane®), and b) at least one other agent that inhibits a prosurvival and/or inflammatory signal, and c) instructions for administering the nanoparticles and the other agents simultaneously, sequentially, and/or concurrently, for the effective treatment of a proliferative disease (such as cancer).

The nanoparticles and the other agents can be present in separate containers or in a single container. It is understood that the medicine may comprise one distinct composition or two or more compositions wherein one composition comprises nanoparticles and one composition comprises another agent.

The kits, medicines, and compositions may include any one or more aspects or parameters described herein.

The invention will now be described in greater detail by reference to the following non-limiting examples. The following examples further illustrate the invention but, of course, should not be construed as in any way limiting its scope.

### EXAMPLES

### Example 1. Induction of Survival and Inflammatory Signals by Chemotherapy in MDA-MB-231 Tumor Cells

Cultured MDA-MB-231 breast tumor cells were treated with 0, 2.5, 5, 10, and 30 nM of Abraxane® for 48 hr. Cell lysates was detected for prosurvival signals (p-STAT3, p42 & p44 kinase, p-NF-κB p65, p-NF-κB p50, p-Akt, bcl-2) using Western blotting. Conditioned media was analyzed for secreted levels of angiogenic (VEGF-A) and inflammatory (IL-6, IL-8, and TNF-a) proteins by ELISA.

Cultured MDA-MB-231 cells were treated with 0, 2.5, 5, 10, and 30 nM of Abraxane® followed by detection of angiogenic (VEGF-A), prosurvival (p42 & p44 kinase, bcl-2) and inflammatory (IL-6, IL-8, and TNF-a) proteins using Western blotting and ELISA.

*In vitro,* Abraxane® treatment increased expression of VEGF-A, p42/44 kinase, bcl-2 as well as total and phosphorylated p65 subunit of NF-kB. Treated cells secreted 25- to 30-fold higher concentrations of inflammatory cytokines IL-6, IL-8, and TNF-a into conditioned media as compared with untreated control cells.

In response to low concentration Abraxane® treatment, pro-survival signals were upregulated in MDA-MB-231 breast tumor cells, including the phosphorylation of p42/p44 kinase, Akt, plus NF-κB p50 and p65 subunits and the expression of bcl-2.

In response to low concentration Abraxane® exposure, increased secretion of VEGF and 25- to 30-fold higher concentrations of inflammatory cytokines IL-6, IL-8, and TNF-a was observed in conditioned media as compared with untreated control cells.

Upregulation of pro-survival and inflammatory signals including VEGF-A, bcl-2, IL-6, IL-8, and TNF-a, as well as phosphorylated NF-κB p65 and p42/44 kinase were seen *in vitro* in response to low dose Abraxane® treatment.

### Example 2. Induction of Survival Signals by Chemotherapy in MDA-MB-231 Breast Tumor Xenografts in vivo

Luciferase-tagged MDA-MB-231 cells were implanted orthotopically into the mammary fatpad of female SCID mice and allowed to reach 500 mm³ in size before treated with 30 mg/kg Abraxane®, IV, qdx5.

Mice were sacrificed 3, 5 or 8 days post treatment and MDA-MB-231 tumors were extracted. Tumor lysates were analyzed for VEGF expression and pro-survival signals (p42 & p44 kinase, p-NF-κB p50, p-Akt, bcl-2) using Western blotting. Expression of bcl-2 was further analyzed by immunohistochemistry.

MDA-MB-231 tumors were extracted from mice upon cessation of intravenous (IV) Abraxane® therapy (10 to 30 mg/kg, qdx5) followed by Western blot and immunohistochemical analyses.

Significant increases in bcl-2 and inflammatory cytokines were observed in tumors extracted immediately after paclitaxel therapy *in vivo* as confirmed by both Western blotting and immunohistochemical analyses.

In the surviving portion of tumors following Abraxane® treatment, there was increased pro-survival signals including the phosphorylation of p42/p44 kinase, Akt, plus NF-κB p50 subunit and the expression of bcl-2. Consistent with previous results (4), VEGF expression was upregulated in the remaining tumors after Abraxane® chemotherapy.

Consistent with *in vitro* results, increase in the expression of VEGF, bcl-2, and activation of pro-survival signaling were noted *in vivo* in the surviving MDA-MB-231 xenograft breast tumors following Abraxane® treatment.

### Example 3. Activation of the NF-κB pathway by nab-paclitaxel in cultured 231-Luc+ cells

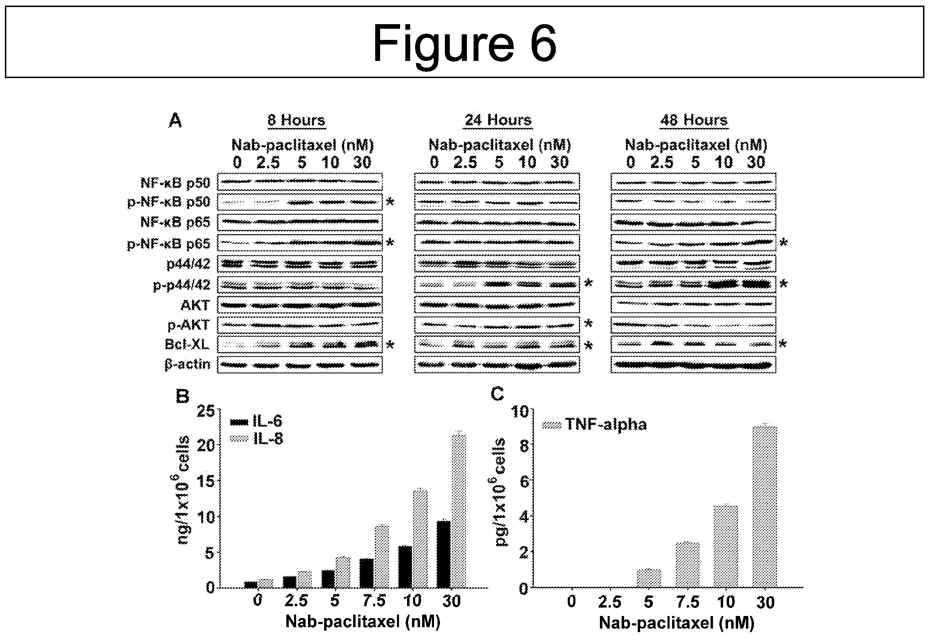

231-Luc+ cells were treated with escalating doses of *nab*-paclitaxel (0-30 nM) for 8-48 hours followed by Western blot analysis. Eight hours after exposure to *nab*-paclitaxel the expression of phosphorylated p-p50 and p-p65 subunits of the NF-κB as well as Bcl-xL was significantly increased (Fig.6A). Later time-points (24h and 48h) showed significant increases in p-Akt and p-p44/42 although the expression of non-phosphorylated counterparts remained unchanged (Fig.6A). NF-κB activation by *nab*-paclitaxel was further confirmed by measuring inflammatory cytokines IL-6 and IL-8, the downstream products of this pathway. Cytokines from conditioned medium of 231-Luc+ cells treated for 72h with *nab*-paclitaxel (2.5-30nM) were measured using Luminex. *Nab*-paclitaxel significantly increased expression of both IL-6 and IL-8 in a dose-dependent manner up to maximum of 20-22-fold (Fig.6B). TNF-a was also increased from undetectable level to 9.5x103 pg normalized per 1x106 cells (Fig.6C). These findings demonstrate that *nab*-paclitaxel activates the NF-κB pathway in tumor cells leading to increased expression of IL-6, IL-8, TNF-a, Bcl-2 and Bcl-xL, all of which may trigger reactionary angiogenesis and promote survival of tumor cells.

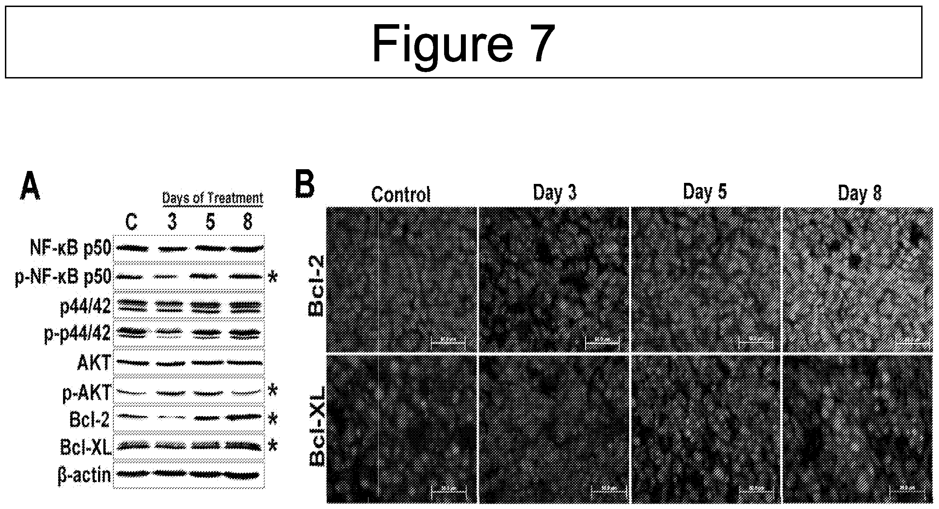

We next determined whether *nab*-paclitaxel behaves similarly *in vivo.* Groups of 231-Luc+ bearing mice (n=4) with advanced tumors (500mm3) were treated with *nab*-paclitaxel (30mg/kg) followed by analysis on the third, fifth and eighth day after initiation of the treatment. Tumor lysates and sections were analyzed for the expression of NF-κB and pro-survival proteins using Western blot and immunohistochemistry, respectively. The expression of several inflammatory and pro-survival proteins including p-p50, p-p44/42, p-Akt, Bcl-2 and Bcl-xL has significantly increased following *nab*-paclitaxel treatment. The highest increase for most targets was on the 8th day post-treatment (Fig.7A). Of all targets, upregulation of Bcl-2 and Bcl-xL was the most conspicuous, and these targets were confirmed by immunohistochemistry. Control tumors displayed homogenous but weak Bcl-2 expression, and strong but sporadically expressed Bcl-xL (Fig.7B, Control). Three days after nab-paclitaxel treatment, the expression of both proteins was slightly decreased or unchanged. However, at 5th and 8th days post-treatment, the expression of both proteins was significantly increased (Fig.7B). Collectively, these data suggest that chemotherapy-induced activation of the NF-κB pathway play an important role in chemoresistance of advanced tumors.

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention.

## Claims

1. A composition comprising nanoparticles comprising a taxane and a carrier protein, for use in a method for treating a proliferative disease, wherein the method comprises the use of said composition in combination with at least one other agent selected from the group consisting of Akt inhibitor, inhibitor of bcl-2, inhibitor of bcl-xL, and inhibitor of both bcl-2 and bcl-xL.

2. The composition for use according to claim 1, wherein said other agent is an Akt inhibitor selected from the group consisting of perifosine, 4-[2-(4-Amino-1,2,5-oxadiazol-3-yl)-1-ethyl-7-[(3*S*)-3-piperidinylmethoxy)-1*H*-imidazo[4,5-c]pyridin-4-yl]-2-methyl-3-butyn-2-ol (GSK690693), triciribine phosphate monohydrate, API-2/TCN, and 1-(3-(4-(3-bromo-1H-pyrazolo[3,4-d]pyrimidin-4-yl)piperazin-1-yl)-4-methyl-5-((2-(pyrrolidin-1-yl)ethyl)amino)phenyl)-4,4,4-trifluorobutan-1-one (XL418).

3. The composition for use according to claim 2, wherein said other agent is perifosine.

4. The composition for use according to claim 3, wherein the method further comprises administering an antimetabolite.

5. The composition for use according to any one of claims 1-4, wherein said Akt inhibitor is in an amount effective to inhibit taxane-mediated upregulation of Akt.

6. The composition for use according to claim 1, wherein the inhibitor of bcl-2 is a small molecule.

7. The composition for use according to claim 6, wherein said small molecule is selected from the group consisting of (R)-ethyl 2-amino-6-bromo-4-((R)-1-cyano-2-ethoxy-2-oxoethyl)-4H-chromene-3-carboxylate (HA 14-1), obatoclax, 4-[4-[[2-(4-chlorophenyl)phenyl]methyl]piperazin-1-yl]-N-[4-[[(2R)-4-(dimethylamino)-1-phenylsulfanylbutan-2-yl]amino]-3-nitrophenyl]sulfonylbenzamide (ABT-737), and navitoclax (ABT-263).

8. The composition for use according to claim 1, wherein the inhibitor of bcl-2 is an antisense oligonucleotide.

9. The composition for use according to claim 8, wherein the antisense oligonucleotide is oblimersen.

10. The composition for use according to claims 8 or 9, wherein the method further comprises administering an alkylating agent.

11. The composition for use according to any one of claims 1-10, wherein the proliferative disease is cancer.

12. The composition for use according to claim 11, wherein the cancer is selected from the group consisting of breast cancer, lung cancer and colorectal cancer.

13. The composition for use according to any one of claims 1-12, wherein the composition comprising nanoparticles comprising taxane and the carrier protein and the other agent are administered simultaneously, sequentially or concurrently.

14. The composition for use according to any one of claims 1-13, wherein the taxane is paclitaxel.

15. The composition for use according to any one of claims 1-14, wherein the average diameter of the nanoparticles in the composition is no greater than about 200 nm.

16. The composition for use according to any one of claims 1-15, wherein the carrier protein is albumin.

17. The composition for use according to claim 16, wherein the weight ratio of the albumin and the taxane in the nanoparticle composition is about 1:1 to about 9:1.

18. A kit or a medicine comprising: a) a composition comprising nanoparticles comprising a taxane and a carrier protein, and b) at least one other agent selected from the group consisting of Akt inhibitor, inhibitor of bcl-2, inhibitor of bcl-xL, and inhibitor of both bcl-2 and bcl-xL.

## Patentansprüche

1. Zusammensetzung, die Nanopartikel umfasst, die ein Taxan und ein Trägerprotein umfassen, zur Verwendung in einem Verfahren zur Behandlung einer proliferativen Erkrankung, wobei das Verfahren die Verwendung der Zusammensetzung in Kombination mit zumindest einem anderen Mittel ausgewählt aus der Gruppe, bestehend aus Akt-Hemmer, Hemmer von bcl-2, Hemmer von bcl-xL, und Hemmer von sowohl bcl-2 und bcl-xL umfasst.

2. Zusammensetzung zur Verwendung nach Anspruch 1, wobei das andere Mittel ein Akt-Hemmer ist, ausgewählt aus der Gruppe, bestehend aus Perifosin, 4-[2-(4-Amino-1,2,5-oxadiazol-3-yl)-1-ethyl-7-[(3S)-3-piperidinylmethoxy]-1H-imidazo[4,5-c]pyri¬din-4-yl]-2-methyl-3-butyn-2-ol (GSK690693), Triciribin-Phosphat-Mono¬hydrat, API-2/TCN und 1-(3-(4-(3-Brom-1H-pyrazolo[3,4-d]pyrimidin-4-yl)pipera¬zin-1-yl)-4-methyl-5-((2-(pyrrolidin-1-yl)ethyl)amino)phenyl)-4,4,4-trifluorbutan-1-on (XL418).

3. Zusammensetzung zur Verwendung nach Anspruch 2, wobei das andere Mittel Perifosin ist.

4. Zusammensetzung zur Verwendung nach Anspruch 3, wobei das Verfahren ferner Verabreicherung eines Antimetaboliten umfasst.

5. Zusammensetzung zur Verwendung nach einem der Ansprüche 1-4, wobei der Akt-Hemmer in einer Menge vorliegt, die wirksam ist, um die Taxan-vermittelte Hochregulierung von Akt zu hemmen.

6. Zusammensetzung zur Verwendung nach Anspruch 1, wobei der Hemmer von bcl-2 ein kleines Molekül ist.

7. Zusammensetzung zur Verwendung nach Anspruch 6, wobei das kleine Molekül ausgewählt ist aus der Gruppe, bestehend aus (R)-Ethyl 2-amino-6-brom-4-((R)-1-cyano-2-ethoxy-2-oxoethyl)-4H-chromen-3-carboxylat (HA 14-1), Obatoclax, 4-[4-[[2-(4-Chlorphenyl)phenyl]methyl]piperazin-1-yl]-N-[4-[[(2R)-4-(dimethylamino)-1-phenylsulfanylbutan-2-yl]amino]-3-nitrophenyl]sulfonylbenzamid (ABT-737), und Navitoclax (ABT-263).

8. Zusammensetzung zur Verwendung nach Anspruch 1, wobei der Hemmer von bcl-2 ein Antisense-Oligonukleotid ist.

9. Zusammensetzung zur Verwendung nach Anspruch 8, wobei das Antisense-Oligonukleotid Oblimersen ist.

10. Zusammensetzung zur Verwendung nach den Ansprüchen 8 oder 9, wobei das Verfahren ferner Verabreicherung eines Alkylierungsmittels umfasst.

11. Zusammensetzung zur Verwendung nach einem der Ansprüche 1-10, wobei die proliferative Erkrankung Krebs ist.

12. Zusammensetzung zur Verwendung nach Anspruch 11, wobei der Krebs ausgewählt ist aus der Gruppe, bestehend aus Brustkrebs, Lungenkrebs und kolorektalem Krebs.

13. Zusammensetzung zur Verwendung nach einem der Ansprüche 1-12, wobei die Zusammensetzung, die Nanopartikel umfasst, die Taxan und das Trägerprotein umfassen, und das andere Mittel simultan, aufeinanderfolgend oder gleichzeitig verabreicht werden.

14. Zusammensetzung zur Verabreichung nach einem der Ansprüche 1-13, wobei das Taxan Paclitaxel ist.

15. Zusammensetzung zur Verwendung nach einem der Ansprüche 1-14, wobei der durchschnittliche Durchmesser der Nanopartikel in der Zusammensetzung nicht größer als etwa 200 nm ist.

16. Zusammensetzung zur Verwendung nach einem der Ansprüche 1-15, wobei das Trägerprotein Albumin ist.

17. Zusammensetzung zur Verwendung nach Anspruch 16, wobei das Gewichtsverhältnis von dem Albumin und dem Taxan in der Nanopartikelzusammensetzung etwa 1:1 bis etwa 9:1 ist.

18. Kit oder Medikament umfassend: a) eine Zusammensetzung, die Nanopartikel umfasst, die ein Taxan und ein Trägerprotein umfassen, und b) zumindest ein anderes Mittel ausgewählt aus der Gruppe, bestehend aus Akt-Hemmer, Hemmer von bcl-2, Hemmer von bcl-xL, und Hemmer von sowohl bcl-2 als auch bcl-xL.

## Revendications

1. Composition comprenant des nanoparticules comprenant un taxane et une protéine porteuse, pour utilisation dans un procédé de traitement d'une maladie proliférative, dans laquelle le procédé comprend l'utilisation de ladite composition en combinaison avec au moins un autre agent choisi dans le groupe constitué d'un inhibiteur d'Akt, d'un inhibiteur de bcl-2, d'un inhibiteur de bcl-xL et d'un inhibiteur à la fois de bcl-2 et de bcl-xL.

2. Composition pour utilisation selon la revendication 1, dans laquelle ledit autre agent est un inhibiteur d'Akt choisi dans le groupe constitué de la périfosine, du 4-[2-(4-amino-1,2,5-oxadiazol-3-yl)-1-éthyl-7-[(3S)-3-pipéridinylméthoxy)-1H-imidazo[4,5-c]pyrridin-4-yl]-2-méthyl-3-butyn-2-ol (GSK690693), du phosphate de triciribine monohydraté, de l'API-2/TCN et de la 1-(3-(4-(3-bromo-1H-pyrrazolo[3,4-d]pyrimidin-4-yl)pipérazin-1-yl)-4-méthyl-5-((2-(pyrrolidin-1-yl)éthyl)amino)phényl)-4,4,4-trifluoro-butane-1-one (XL418).

3. Composition pour utilisation selon la revendication 2, dans laquelle ledit autre agent est la périfosine.

4. Composition pour utilisation selon la revendication 3, dans laquelle le procédé comprend en outre l'administration d'un antimétabolite.

5. Composition pour utilisation selon l'une quelconque des revendications 1 à 4, dans laquelle ledit inhibiteur d'Akt est en une quantité efficace pour inhiber la régulation positive de l'Akt médiée par le taxane.

6. Composition pour utilisation selon la revendication 1, dans laquelle l'inhibiteur de bcl-2 est une petite molécule.

7. Composition pour utilisation selon la revendication 6, dans laquelle ladite petite molécule est choisie dans le groupe constitué du (R)-éthyl-2-amino-6-bromo-4-((R)-1-cyano-2-éthoxy-2-oxoéthyl)-4H-chromène-3-carboxylate (HA 14-1), de l'obatoclax, du 4-[4-[[2-(4-chlorophényl)phényl]méthyl]pipérazin-1-yl]-N-[4-[[(2R)-4-(diméthylamino)-1-phénylsulfanylbutan-2-yl]amino]-3-nitrophényl]sulfonylbenzamide (ABT-737) et du navitoclax (ABT-263).

8. Composition pour utilisation selon la revendication 1, dans laquelle l'inhibiteur de bcl-2 est un oligonucléotide antisens.

9. Composition pour utilisation selon la revendication 8, dans laquelle l'oligonucléotide antisens est l'oblimersène.

10. Composition pour utilisation selon les revendications 8 ou 9, dans laquelle le procédé comprend en outre l'administration d'un agent d'alkylation.

11. Composition pour utilisation selon l'une quelconque des revendications 1 à 10, dans laquelle la maladie proliférative est le cancer.

12. Composition pour utilisation selon la revendication 11, dans laquelle le cancer est choisi dans le groupe constitué du cancer du sein, du cancer du poumon et du cancer colorectal.

13. Composition pour utilisation selon l'une quelconque des revendications 1 à 12, dans laquelle la composition comprenant des nanoparticules comprenant du taxane et la protéine porteuse et l'autre agent sont administrés simultanément, séquentiellement ou conjointement.

14. Composition pour utilisation selon l'une quelconque des revendications 1 à 13, dans laquelle le taxane est le paclitaxel.

15. Composition pour utilisation selon l'une quelconque des revendications 1 à 14, dans laquelle le diamètre moyen des nanoparticules de la composition n'est pas supérieur à environ 200 nm.

16. Composition pour utilisation selon l'une quelconque des revendications 1 à 15, dans laquelle la protéine porteuse est l'albumine.

17. Composition pour utilisation selon la revendication 16, dans laquelle le rapport pondéral de l'albumine et du taxane dans la composition de nanoparticules est d'environ 1:1 à environ 9:1.

18. Kit ou médicament comprenant: a) une composition comprenant des nanoparticules comportant un taxane et une protéine porteuse et b) au moins un autre agent choisi dans le groupe constitué d'un inhibiteur d'Akt, d'un inhibiteur de bcl-2, d'un inhibiteur de bcl-xL et d'un inhibiteur à la fois de bcl-2 et de bcl-xL.
